(19) **Europäisches Patentamt European Patent Office Office européen des brevets**

(11) **EP 3 019 592 B1**

(12) **FASCICULE DE BREVET EUROPEEN**

(45) Date de publication et mention de la délivrance du brevet:
**03.07.2019 Bulletin 2019/27**

(51) Int Cl.:
*C12N 1/12* *(2006.01)* *C12N 13/00* *(2006.01)*
*C12P 7/64* *(2006.01)* *C12P 23/00* *(2006.01)*
*C12R 1/89* *(2006.01)*

(21) Numéro de dépôt: **14747099.1**

(22) Date de dépôt: **15.07.2014**

(86) Numéro de dépôt international:
**PCT/FR2014/051808**

(87) Numéro de publication internationale:
**WO 2015/004402 (15.01.2015 Gazette 2015/02)**

(54) **NOUVELLE SOUCHE DE AURANTIOCHYTRIUM ET PROCÉDÉ UTILISANT CELLE-CI**

NEUARTIGER STAMM VON AURANTIOCHYTRIUM UND VERFAHREN ZU DESSEN VERWENDUNG.

NOVEL STRAIN OF AURANTIOCHYTRIUM AND PROCESS USING IT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.07.2013 FR 1356917**

(43) Date de publication de la demande:
**18.05.2016 Bulletin 2016/20**

(83) **Déclaration conformément à la règle 32(1) CBE (solution de l'expert)**

(73) Titulaire: **Fermentalg**
**33500 Libourne (FR)**

(72) Inventeurs:
• **GODART, François**
**33870 Vayres (FR)**
• **PAGLIARDINI, Julien**
**33300 Bordeaux (FR)**
• **ROLS, Cyril**
**13004 Marseille (FR)**
• **CALLEJA, Pierre**
**33500 Libourne (FR)**

(74) Mandataire: **Be IP**
**Cabinet LTL SAS**
**Centre d'Entreprise et d'Innovation**
**56, Bd Niels Bohr**
**CS 52132**
**69603 Villeurbanne Cedex (FR)**

(56) Documents cités:
**EP-A1- 2 559 342** **WO-A1-2012/035262**
**WO-A1-2013/136028** **US-A1- 2009 209 014**

• **CHATDUMRONG W ET AL: "Optimization of docosahexaenoic acid (DHA) production and improvement of astaxanthin content in a mutant Schizochytrium limacinum isolated from mangrove forest in Thailand", KASETSART JOURNAL (NATURAL SCIENCE), KASETSART UNIVERSITY, THAILAND , vol. 41 1 janvier 2007 (2007-01-01), pages 324-334, XP002688962, ISSN: 0075-5192 Extrait de l'Internet: URL:http://kasetsartjournal.ku.ac.th/kuj_f iles/2008/A0804171228243424.pdf [extrait le 2012-12-10]**
• **WON-KYUNG HONG ET AL: "Production of Lipids Containing High Levels of Docosahexaenoic Acid by a Newly Isolated Microalga,sp. KRS101", APPLIED BIOCHEMISTRY AND BIOTECHNOLOGY ; PART A: ENZYME ENGINEERING AND BIOTECHNOLOGY, HUMANA PRESS INC, NEW YORK, vol. 164, no. 8, 22 mars 2011 (2011-03-22) , pages 1468-1480, XP019922295, ISSN: 1559-0291, DOI: 10.1007/S12010-011-9227-X**

EP 3 019 592 B1

**(Cont. page suivante)**

- **ANITA N JAKOBSEN ET AL: "Accumulation of docosahexaenoic acid-rich lipid in thraustochytrid Aurantiochytrium sp. strain T66: effects of N and P starvation and O2 limitation", APPLIED MICROBIOLOGY AND BIOTECHNOLOGY, SPRINGER, BERLIN, DE, vol. 80, no. 2, 17 juin 2008 (2008-06-17), pages 297-306, XP019623690, ISSN: 1432-0614**
- **RINKA YOKOYAMA ET AL: "Taxonomic rearrangement of the genus Schizochytrium sensu lato based on morphology, chemotaxonomic characteristics, and 18S rRNA gene phylogeny (Thraustochytriaceae, Labyrinthulomycetes): emendation for Schizochytrium and erection of Aurantiochytrium and Oblongichytrium gen. nov", MYCOSCIENCE, SPRINGER-VERLAG, TO, vol. 48, no. 4, 6 août 2007 (2007-08-06), pages 199-211, XP019521654, ISSN: 1618-2545, DOI: 10.1007/S10267-006-0362-0 cité dans la demande**

## Description

L'invention se rapporte à un procédé de culture en mode mixotrophe, notamment en présence d'un éclairement discontinu et/ou variable de lumière, d'un protiste de la classe *Labyrinthulomycete*, en particulier du genre *Aurantiochytrium.* Le procédé permet d'obtenir un haut rendement en biomasse et un enrichissement des protistes ainsi cultivés en lipides et en caroténoïdes, et plus particulièrement en acide docosahexaénoïque (DHA) et en canthaxanthine et/ou en astaxanthine. Le procédé permet ainsi de cultiver des souches du genre *Aurantiochytrium* à caractère mixotrophe, et ayant un haut rendement en lipides et/ou caroténoïdes, et plus particulièrement en acides gras polyinsaturés et en canthaxanthine et/ou en astaxanthine. L'invention se rapporte aussi à de nouvelles souches de protiste appartenant au genre *Aurantiochytrium,* particulièrement adaptée à la production de lipides et de caroténoïdes. Ces nouvelles souches du genre *Aurantiochytrium* sont utiles pour produire de l'acide docosahexaénoïque (DHA) et de la canthaxanthine et/ou de l'astaxanthine en mode mixotrophe.

## Préambule

Parmi les acides gras polyinsaturés, certains hautement insaturés (AGHI) de la série des oméga-3 (PUFA-$\omega$3), en particulier l'acide éicosapentaénoïque (EPA ou C20:5 $\omega$3) et l'acide docosahexaénoïque (DHA ou C22:6 $\omega$3), et de la série des oméga-6 (PUFA-$\omega$6), en particulier, l'acide arachidonique (ARA ou AA ou encore acide eicosatétraénoïque C20:4 $\omega$6) ont une importance nutritionnelle reconnue et présentent de fortes potentialités en terme d'applications thérapeutiques.

Considéré comme nutriment essentiel, le DHA est nécessaire au développement normal et fonctionnel des cellules, et joue un rôle crucial dans divers processus et fonctions biochimiques. Sa nature polyinsaturée lui confère une importance cruciale vis-à-vis des propriétés de la membrane cellulaire, chez les végétaux comme chez les animaux : fluidité, flexibilité et perméabilité sélective permettant par exemple une adaptation effective, et même la survie, aux basses températures, en particulier chez les poissons.

Le DHA est un constituant structurel majeur du cerveau humain et il en est le principal acide gras. Le DHA représente 15-20 % du cortex cérébral (le cerveau d'un adulte contient au moins 20 g de DHA) et 30-60 % de la rétine. Il est indispensable au développement du système nerveux central et à la fonction rétinienne, par incorporation dans les membranes cellulaires, et joue un rôle capital dans l'acquisition et le maintien satisfaisant des mécanismes de la vision et de la mémoire.

Les huiles de poissons, issues de l'industrie de la pêche, sont actuellement la principale source commerciale de ce type d'acides gras. Toutefois, alors que ces huiles trouvent de nouvelles applications (complément alimentaire en aquaculture, intégration dans les margarines), les ressources halieutiques marines se raréfient du fait d'une activité de pêche intensive.

De nouvelles sources de ces acides gras tels que l'EPA, le DHA et l'ARA doivent donc être recherchées afin de répondre, dans le futur, à la demande croissante du marché pour ce type d'acides gras polyinsaturés.

Les protistes sont des microorganismes à organisation cellulaire simple, c'est-à-dire qu'ils sont généralement unicellulaires et parfois multicellulaires mais ne présentent pas de tissus spécialisés. Ils peuvent être autotrophes ou hétérotrophes.

Les protistes font l'objet actuellement de nombreux projets industriels car certaines espèces sont capables d'accumuler ou de secréter des quantités importantes de lipides, notamment d'acides gras polyinsaturés. On citera notamment les procédés décrits dans US 2009/209014 et la sélection de mutants décrite par Chatdumrong & al. (KasetsartJ. (Nat.Sci.) 41(2) 324-334, 2007).

Outre leur capacité à synthétiser les acides gras *de novo,* les protistes offrent plusieurs avantages par rapport aux huiles de poisson : elles sont cultivables *in vitro* dans des conditions contrôlées, ce qui permet la production d'une biomasse de composition biochimique relativement constante et, d'autre part, contrairement aux huiles de poissons, leurs lipides ne contiennent pas ou peu de cholestérol.

Enfin, les lipides produits par les protistes ont un profil d'acides gras plus simple que celui des huiles de poissons, ce qui limite les étapes de séparation des acides gras d'intérêt.

Par ailleurs, les caroténoïdes sont également des molécules d'intérêt. Ils sont généralement utilisés en tant que pigments, mais ils ont aussi un rôle important pour la santé de l'homme en tant qu'agents antioxydants. Enfin, ils présentent la capacité de stimuler le système immunitaire.

Les Thraustochytrides, en particulier *Aurantiochytrium,* sont connus pour produire du DHA lorsqu'ils sont cultivés en hétérotrophie [W.K. Hong et al. (2011); Production of lipids containing high levels of docosahexaenoic acid by a newly isolated microalga, Aurantiochytrium sp. KRS101. Appl. Biochem. Biotechnol.: 164(8) :1468-80]. *Aurantiochytrium* est aussi connue pour produire des caroténoïdes, tels que l'astaxanthine, la zéaxanthine, la canthaxanthine, l'échinénone, le bêta-carotène et la phoenicoxanthine [Yokoyama, R, Honda, D. (2007) Taxonomic rearrangement of the genus Schizochytrium sensu lato based on morphology, chemotaxonomic characteristics, and 18S rRNA gene phylogeny (Thraustochytriaceae, Labyrinthulomycetes): emendation for Schizochytrium and erection of Aurantiochytrium and Oblongichytrium gen. nov.; Mycoscience, Vol. 48, pp 199-211].

Pour mettre en oeuvre la production des acides gras et des caroténoïdes par des protistes à l'échelle industrielle, plusieurs facteurs doivent être pris en compte. Par exemple, les cultures peuvent être réalisées en condition autotrophe, mixotrophe ou hétérotrophe selon la souche, la température, les conditions de lumière et la taille des fermenteurs. Par exemple, les cultures peuvent également être réalisées dans des conteneurs d'un litre, dans un laboratoire, dans des photobioréacteurs, et dans des conteneurs de 100 000 litres ou bien dans des bassins ouverts (plusieurs hectares). Toutefois, les dépenses énergétiques et autres ressources telles que la main d'oeuvre et la facilité de poursuivre la culture doivent être prises en compte en développant des conditions de culture idéales.

En tout état de cause, il serait souhaitable de pouvoir obtenir des rendements en DHA et en caroténoïdes plus importants que ce qui est décrit dans l'art antérieur pour une exploitation industrielle plus efficace et plus rentable.

Ainsi, il est préférable d'avoir un rendement le plus élevé possible (par exemple au-delà de 80 g/l de matière sèche, plus de 30% d'acides gras en poids par rapport au poids total de la matière sèche et plus de 0,1 % en poids de caroténoïdes sur le poids total de matière sèche).

L'effet de la lumière sur la croissance n'est pas réservé uniquement aux organismes photosynthétiques (plantes, cyanobactéries, microalgues et macroalgues). Certains organismes non chlorophylliens possèdent des photorécepteurs qui leur permettent de capter l'énergie lumineuse, essentielle pour leur développement. Les photorécepteurs comme le phytochrome représentent un exemple typique d'un capteur de lumière qui contrôle le développement de l'organisme qui le possède. Différents photorécepteurs ont été décrits à ce jour, à savoir, entre autres, les pigments accessoires, les cryptochromes et phototropines.

De plus, concernant les modes de cultures il existe trois types de procédés de fermentation couramment utilisés dans le domaine de la production de biomasse.

- le mode discontinu (en anglais « batch ») :

La cuve est remplie par le milieu de culture stérilisé, puis l'inoculum. La fermentation se déroule ensuite sans addition majeure supplémentaire de milieu (principalement les correctifs pH et antimousse). Le volume reste constant et la productivité de biomasse est relativement faible. En fin de fermentation, le fermenteur est vidé et son contenu est remplacé.

- le mode semi-continu (en anglais « fed-batch ») :

Ce procédé de production repose sur l'alimentation progressive de substrat nutritif lors de la culture. Ce mode est généralement utilisé pour atteindre une densité cellulaire élevée dans le fermenteur.

- le mode continu (en anglais « continuous ») :

Dans ce procédé, on utilise un fermenteur couramment appelé « chemostat » dans lequel un milieu de culture frais est ajouté en permanence, tandis que le liquide de culture est soutiré en permanence pour garder le volume de culture constant.

Le taux de croissance des cellules peut être contrôlé en modifiant le débit d'alimentation en matière nutritive tout en respectant les limites physiologiques de chaque type de cellule ou souche (en fonction de son taux de croissance spécifique maximal ($\mu$max) dans les conditions spécifiques d'un procédé).

Le mode continu permet la culture des microorganismes dans un état d'équilibre physiologique donné correspondant à un équilibre entre la croissance de la biomasse et la production de métabolite(s) d'intérêt.

Dans l'état d'équilibre, les cellules croissent à une vitesse constante et tous les paramètres de culture demeurent constants (pH, volume, concentration en oxygène dissous, concentrations en éléments nutritifs et en produits, densité cellulaire, etc..).

Le mode continu présente, en particulier, les avantages suivants :

- le coût de production global de biomasse est diminué (moins d'opérations de nettoyage, stérilisation, culture, immobilisation d'équipements au niveau de la fermentation);
- la productivité (quantité de biomasse produite par heure) est augmentée;
- la récupération de la biomasse de manière continue permet de faciliter les opérations en aval tout en optimisant le dimensionnement des équipements.

Cependant, les procédés continus présentent également des inconvénients, notamment lorsque la production de la molécule d'intérêt est dissociée ou partiellement dissociée de la phase de croissance cellulaire. Ainsi, lorsqu'une substance d'intérêt est produite en dehors de cette phase de croissance optimale (par exemple en fin de culture, pendant la phase stationnaire de la culture), il est plus difficile d'obtenir des performances compatibles avec les intérêts économiques.

D'autres contraintes liées au mode continu sont :

- la gestion des effluents en plus grande quantité (concentration en biomasse plus faible),
- la perte de matières premières non consommées (substrats résiduels) pouvant également avoir un impact sur les processus en aval (extraction, séchage...).

Il est, donc souhaitable de réaliser des cultures des souches *d'Aurantiochytrium* pour la production de DHA et/ou de canthaxanthine et/ou d'astaxanthine selon le mode de culture qui va permettre d'atteindre un taux de biomasse et des molécules d'intérêts de manière rentable à l'échelle industrielle,

Pour améliorer le rendement en DHA et en caroténoïdes, en particulier de la canthaxanthine et/ou en astaxanthine, le procédé de culture selon l'invention est pratiqué en mode mixotrophe, c'est-à-dire avec un apport de lumière, de préférence, avec une intensité variable et/ou discontinue et en présence d'un apport de substrat organique.

Il est entendu que le terme mixotrophe est usuellement employé à l'égard de souches présentant un chloroplaste, capable de se développer en présence de lumière en utilisant deux sources de carbone (organique et inorganique). Dans le cas des souches du genre *Aurantiochytrium,* un tel chloroplaste n'a pas été identifié. Toutefois, la souche étant à la fois hétérotrophe et réactive à la lumière, le terme mixotrophe sera élargi au sens de l'invention à cette catégorie de souche. On peut également utiliser le terme « photo-hétérotrophe » pour indiquer une telle souche.

Ainsi, c'est au terme de nombreuses expérimentations de criblage de souches que le demandeur est parvenu à isoler des souches de protiste du genre *Aurantiochytrium,* cultivables en mode mixotrophe et permettant, dans les conditions de la présente invention, une production à haut rendement d'acides gras polyinsaturés et de caroténoïdes, notamment de DHA et des caroténoïdes, notamment de la canthaxanthine et de l'astaxanthine.

Des souches représentatives des nouvelles souches *d'Aurantiochytrium* ainsi isolées et sélectionnées, ont été déposées auprès de la CCAP (Culture Collection of Algae and Protozoa, Scottish Association for Marine Science, Dunstaffnage Marine Laboratory, Oban, Argyll PA371QA, Ecosse, Royaume-Uni).

Ainsi la souche (FCC 1324) a été déposée le 21 juin 2013 selon les dispositions du Traité de Budapest, sous le numéro d'accession CCAP 4062/1 auprès de la CCAP.

Les souches FCC 31 (numéro d'accession CCAP 4062/2) ; FCC 1311 (numéro d'accession CCAP 4062/3) ; FCC 1319 (numéro d'accession CCAP 4062/4) ; FCC 1325 (numéro d'accession CCAP 4062/5) et FCC 1479 (numéro d'accession CCAP 4062/6), ont été déposées le 8 juillet 2014 selon les dispositions du Traité de Budapest, auprès de la CCAP.

Selon un mode de réalisation, la culture en mode mixotrophe se fait dans des conditions d'éclairement discontinu et/ou variable au cours du temps, l'éclairement présentant des variations d'intensité dont l'amplitude est comprise entre 5 $\mu$mol. m$^{-2}$. s$^{-1}$ et 1000 $\mu$mol. m$^{-2}$. s$^{-1}$, ces variations ayant lieu entre 2 et 3600 fois par heure. L'amplitude des variations d'intensité est comprise entre 70 et 300 $\mu$mol. m$^{-2}$. s$^{-1}$, et de préférence, entre 100 et 200 $\mu$mol. m$^{-2}$. s$^{-1}$.

**Légendes des figures** :

**Figure 1.** Schéma de procédé selon un mode de réalisation de l'invention, le « procédé découplé ».

**Figure 2. Comparaison de croissance entre la souche FCC 1324 (CCAP 4062/1) et la souche FCC 1104 *d'Aurantiochytrium mangrovei* (numéro de dépôt CCAP4087/1).** (A) Croissance en présence de NH$_4^+$ comme source d'azote. (B) Croissance en présence de NO$_3^-$ comme source d'azote.

**Figure 3. Images de microscopie photonique cellules ayant poussé en milieu contenant du NH$_4^+$ comme source d'azote.** (A) image de la souche FCC1324 (CCAP 4062-1). (B) Image de la souche FCC1104 *d'Aurantiochytrium mangrovei* (CCAP4087/1). Les deux images ont été prises avec le même grossissement x1000.

**Description détaillée**

La présente invention a donc pour objet un procédé de production de DHA et/ou de canthaxanthine et/ou de l'astaxanthine, comprenant l'étape suivante :

a) la culture en mode mixotrophe d'une ou plusieurs souches du genre *Aurantiochytrium* caractérisé en ce que

l'étape a) de la culture comprend

- Une étape i) de croissance en conditions de mixotrophie ou d'hétérotrophie et
- Une étape ii) d'accumulation de DHA et/ou de caroténoïdes en conditions de mixotrophie à une température entre 15 et 25°C,

caractérisé en ce que la souche du genre *Aurantiochytrium* correspond à la souche FCC 1324, déposée auprès de la CCAP (Culture Collection of Algae and Protozoa), sous le numéro d'accession CCAP 4062/1 et l'étape ii)se fait à une température plus basse de 1 à 8 degrés que l'étape i).

L'éclairement présente préférentiellement des variations d'intensité dont l'amplitude est généralement comprise entre 5 $\mu$mol. m$^{-2}$. s$^{-1}$ et 1000 $\mu$mol. m$^{-2}$. s$^{-1}$, de préférence entre 30 et 400 $\mu$mol. m$^{-2}$. s$^{-1}$. Selon un mode de réalisation de l'invention ces variations peuvent avoir lieu entre 2 et 3600 fois par heure, de préférence entre 2 et 200 fois par heure. Cet apport lumineux peut comporter des phases d'éclairement discontinu et/ou variable, avec des variations d'intensité pouvant avoir des amplitudes identiques ou différentes.

Selon un mode de réalisation l'apport de lumière s'effectue sous forme de flashs. Selon un mode de réalisation un flash a une durée comprise entre 1/10 seconde et 10 minutes, de préférence 5 secondes et 10 minutes, de préférence encore entre 10 secondes et 2 minutes, plus préférentiellement entre 20 secondes et 1 minute.

Selon un mode de réalisation, le nombre de flashs est entre 5 et 3600, de préférence entre 10 et 150, préférentiellement entre 15 et 100, et plus préférentiellement entre 20 et 50 fois par heure.

Selon un mode de réalisation, l'éclairement des cultures est assuré par un dispositif d'illumination interne au fermenteur, de préférence, situé sur les contre-pales à l'intérieur du fermenteur. Selon un mode de réalisation préféré des sources d'éclairement sont des diodes électro-luminescentes.

Selon un mode de réalisation de l'invention, le procédé de culture en mode mixotrophe s'effectue en présence de 100 mM à 1,5 M, de préférence de 200 mM à 1,2 M, plus préférentiellement de 250 mM à 1 M, et encore plus préférentiellement de 300 mM à 600 mM d'un substrat carboné organique. L'apport du substrat est assuré continuellement pendant la culture, afin de permettre aux cellules d'accumuler une concentration importante de lipides et de caroténoïdes. Du substrat additionnel est ajouté au milieu de culture pendant le procédé de culture pour maintenir une concentration suffisante.

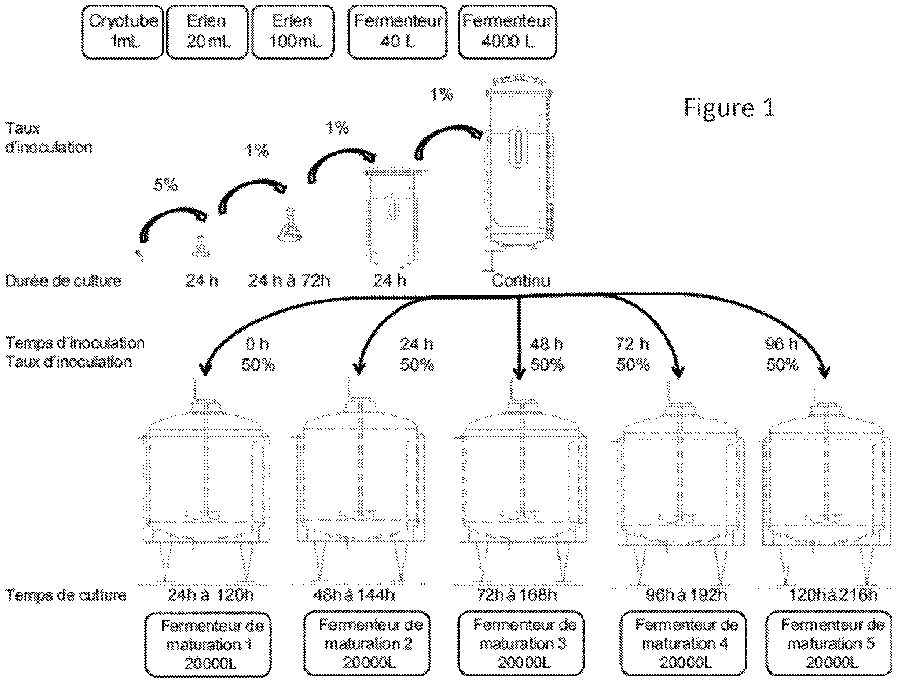

Selon un autre mode de réalisation de l'invention, le procédé de culture comprend la culture des cellules en mode continu en conditions de mixotrophie ou en hétérotrophie dans un fermenteur (étape de « croissance »), puis l'alimentation en continu dans des fermenteurs fonctionnant en mode semi-continu par les cellules produites et leur culture en conditions de mixotrophie dans lesdits fermenteurs (étape de « maturation ») (voir **Figure 1**).

Le procédé selon l'invention a pour avantage d'augmenter le rendement en biomasse obtenu de la culture. Il a aussi pour avantage d'enrichir les protistes ainsi cultivés en acides gras polyinsaturés, plus particulièrement en acide docosahexaénoïque (DHA), et en caroténoïdes.

La culture en mode mixotrophe de ce protiste s'effectue préférentiellement en présence de 100 mM à 1,5 M, de préférence de 200 mM à 1,2 M, plus préférentiellement de 250 mM à 1 M, et encore plus préférentiellement de 300 mM à 600 mM d'un substrat carboné organique. L'apport du substrat est préférentiellement assuré continuellement pendant la culture, afin de permettre aux cellules d'accumuler une concentration importante de lipides et de caroténoïdes. Du substrat additionnel est ajouté au milieu de culture pendant le procédé de culture pour maintenir une concentration suffisante. Ce substrat carboné organique comprend préférentiellement, sous forme pure ou en mélange : du glucose, des dérivés de cellulose, du saccharose et/ou du glycérol.

Le substrat carboné organique contenu dans le milieu de culture peut consister en des molécules complexes ou en un mélange de substrats. Les produits issus de la biotransformation de l'amidon, par exemple à partir de maïs, de blé ou de pomme de terre, notamment les hydrolysats de l'amidon, qui sont constitués de molécules de petite taille, constituent, par exemple, des substrats carbonés organiques adaptés à la culture en mixotrophie des protistes selon l'invention.

Selon un mode de réalisation, ledit substrat carboné organique présent dans le milieu de culture comprend au moins 300 mM de glucose et/ou glycérol.

Par « conditions de mixotrophie », on entend une culture avec un apport de lumière et un apport en substrat carboné organique.

Par « conditions en hétérotrophie », on entend un apport en substrat carboné organique et dans l'obscurité.

Certaines souches appartenant à l'espèce *Aurantiochytrium mangrovei* présentent des différences entre elles. Les souches CCAP 4062/1 et une souche d'*Aurantiochytrium mangrovei* FCC 1104 (numéro de dépôt CCAP4087/1) décrite dans EP 2 559 342se distinguent au niveau de leurs caractéristiques morphologiques et leurs profils de croissance.

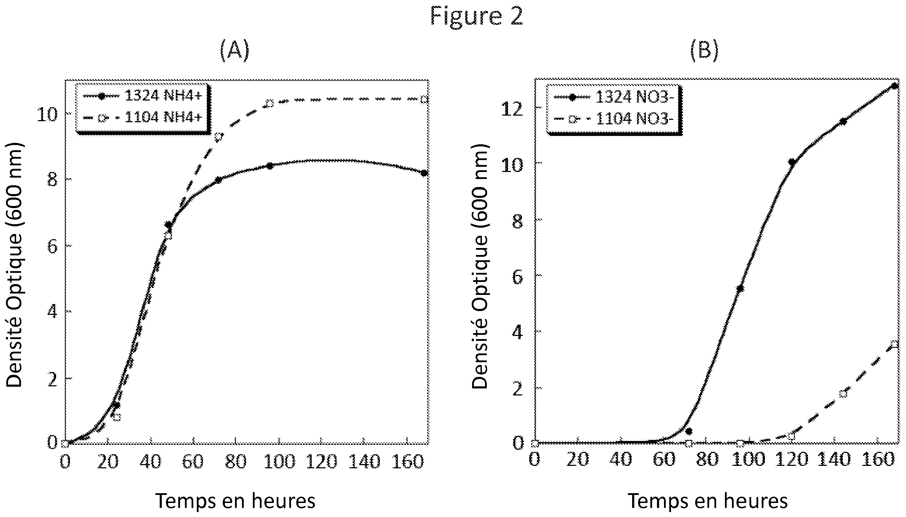

Les inventeurs ont pu voir la distinction par des tests de croissance et des analyses morphologiques. Dans les mêmes conditions de culture, la croissance de ces deux souches est différente, tant au niveau de la vitesse de croissance qu'au niveau de la densité optique atteinte lors de la phase de saturation (voir la **Figure 2A** et **2B**). Effectivement, la souche CCAP4087/1 se développe plus sur un milieu contenant du $NH_4^+$ par rapport à la souche CCAP4062/1, alors qu'on observe l'inverse en utilisant un milieu contenant du $NO_3^-$ comme source d'azote.

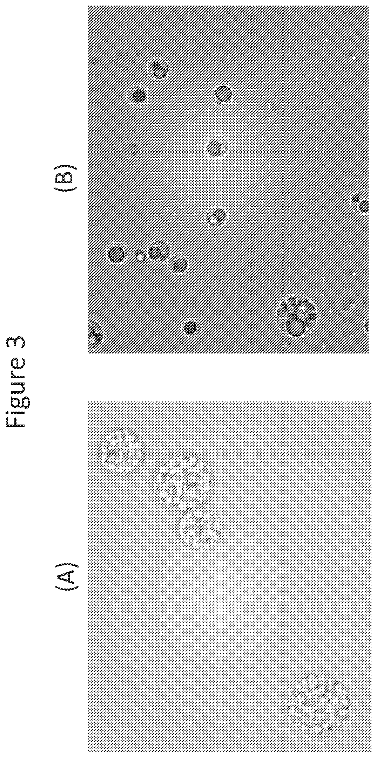

De plus les souches CCAP4087/1 sont plus petites (voir la **Figure 3A** et **3B**).

Selon un mode de réalisation de l'invention, particulièrement adapté à la production de DHA et/ou de caroténoïde(s) à l'échelle industrielle, l'étape i) est réalisée en mode continu dans un fermenteur et l'étape ii) comprend l'alimentation en continu et successivement de *n* fermenteurs fonctionnant en mode semi-continu, *n* étant un nombre entier égal ou supérieur à 2, par les cellules produites dans l'étape i) et leur culture en conditions de mixotrophie.

L'étape i) est la phase de croissance des cellules permettant d'atteindre une population importante. En général, un milieu de culture adapté pour la culture des souches *d'Aurantiochytrium* est utilisé.

L'étape ii) est une phase d'accumulation de DHA et/ou de caroténoïde(s). De préférence, cette phase utilise une seconde solution d'alimentation permettant d'orienter préférentiellement le métabolisme vers l'accumulation de ces molécules d'intérêt. Par exemple, en cas de production de DHA, cette orientation peut se faire par l'augmentation du rapport de la concentration du carbone sur celle de l'azote assimilable (rapport C/N).

En pratique, un premier fermenteur fonctionnant en mode continu permet la croissance (l'étape i)) des cellules, et le flux sortant (ou soutirage) de ce fermenteur de croissance alimente des cuves de maturation où la biomasse s'enrichit en molécules d'intérêt (l'étape ii)).

Selon ce mode de réalisation de l'invention, le procédé peut être décrit en tant qu'un procédé dit « découplé » car les phases de croissance selon l'étape i) et de maturation selon l'étape ii) sont séparées physiquement et temporellement.

L'étape i) de culture des cellules en mode continu du procédé de production de biomasse selon l'invention, ou « étape de croissance », peut être mise en oeuvre dans un fermenteur connu de l'homme de métier, comme par exemple, le « chemostat ».

Le démarrage de la culture de l'étape i) peut se faire de manière classique (voir **Figure 1**). Des étapes de préculture peuvent être effectuées en amont, de manière à obtenir la concentration cellulaire voulue pour débuter la production de biomasse en continu, à savoir entre 2 et 50 g/L, de préférence entre 5 et 40 g/L.

En général, la montée en échelle à partir du cryotube se fait en plusieurs étapes de pré-culture. Par exemple, elle peut se faire en trois étapes, deux étapes en Erlenmeyer (préculture 1 et préculture 2), et une étape en fermenteur (pré-fermenteur). La croissance en Erlenmeyer se déroule en mode discontinu (« batch »). En général, la croissance en pré-fermenteur se fait également en mode discontinu.

Par exemple, la culture peut passer progressivement d'une culture en Erlenmeyer de 20 mL à une culture en Erlenmeyer de 2 L, puis à une culture en fermenteur (pré-fermenteur) en mode discontinu (« batch »), puis, à une culture dans un fermenteur en mode continu (« fermenteur de tête »). Par exemple, le fermenteur en mode discontinu a un volume de 40 L et le fermenteur de tête a un volume de 4000 L.

Le démarrage de la culture dans le fermenteur de tête a une phase de mise en place durant laquelle le régime continu n'est pas atteint, c'est-à-dire que la croissance des cellules n'est pas stabilisée, ou n'est pas en phase avec le débit d'alimentation en milieu frais. Il s'agit d'une phase « d'atteinte de l'équilibre » que l'on pourrait aussi appeler « phase de mise en régime permanent ». Cette « phase de mise en régime permanent » a une durée qui dépend de la vitesse de croissance des microorganismes cultivés ou plus précisément du taux de croissance spécifique ou $\mu$max. A titre exemple, cette durée peut être comprise entre 5 et 200 heures, de préférence entre 10 et 100 heures.

De manière générale, une fois le régime permanent atteint, un débit de soutirage du fermenteur de tête vers le ou les fermenteur(s) de maturation est mis en place à un débit équivalent au débit d'alimentation en milieu de culture frais. Ce soutirage alimente un premier fermenteur de maturation jusqu'à ce que le volume souhaité soit transféré, puis le soutirage est réorienté vers une autre cuve de maturation. L'homme de métier saura déterminer le volume d'inoculum nécessaire selon le type de cellules à cultiver, la molécule d'intérêt à produire et les capacités des cuves de maturation selon un mode de réalisation. Ce volume d'inoculum peut être compris entre 10 et 90 % de la capacité du fermenteur de maturation, en fonction des cellules cultivées ; par exemple environ 20 %, 30 %, 40 %, 50 %, 60 %, 70 %, ou 80 % de la capacité du fermenteur de maturation.

Le soutirage vers les fermenteurs de maturation est fait de manière conventionnelle en utilisant des équipements connus.

De manière générale, un milieu de culture différent de celui utilisé pendant l'étape i) est employé pendant la phase de maturation. Selon des modes de réalisation différents, ce milieu de culture est apporté dans le fermenteur de maturation simultanément, ou préalablement, ou ultérieurement à l'inoculum qui provient du soutirage du fermenteur de tête.

Le volume de la culture en fermenteur de l'étape i) est déterminé en fonction du volume final de culture souhaité pour les cultures en fermenteurs de maturation. En général, le fermenteur de tête a un volume de 5 à 100 %, de préférence 10 % à 50 % et plus préférentiellement environ 20 % du volume du fermenteur de maturation qu'il alimente. Par exemple, un fermenteur de tête ayant un volume de 4000 L peut alimenter un fermenteur de maturation de 20 000 L. Si les n fermenteurs de maturation ont des volumes différents, c'est le volume de fermenteur le plus important qui va déterminer le volume du fermenteur en tête.

De manière générale, la culture selon l'étape i) des souches de protistes du genre *Aurantiochytrium* pour la production de DHA peut se faire en milieu de culture salin (sel marin 10-20 g/L, de préférence 15 g/L) avec une concentration de glucose de 5 à-200 g/L, de préférence 30 à 100 g/L et plus préférentiellement de 30 à 60 g/L. Selon un mode de réalisation, le milieu de culture contient également des macroéléments, tels que des sels de magnésium et des sels de potassium, à une concentration de 1 à 50 g/L, de préférence 2 à 25 g/L. Il contient aussi des oligoéléments utilisés de manière conventionnelle pour la culture des protistes, tels que des sels de manganèse, de zinc, de cobalt, de molybdène et de cuivre, de nickel et de fer. Des valeurs typiques pour la concentration de ces oligoéléments sont de 0,02 à 15 mg/L pour chaque oligoélément. Par exemple, les sels de magnésium et de zinc peuvent être présents à 2-4mg/L, les sels de cobalt et de molybdène peuvent être présents en faible quantité, telle que 0,01-0,04 mg/L, de préférence entre 0,02-0,03 mg/L. Les sels de cuivre et de nickel peuvent être présents à 1- 4 mg/L, de préférence à 1-2 mg/L. En général, les sels de fer sont présents à un taux plus important par rapport à d'autres sels, par exemple à un niveau de 8-15 mg/L.

Selon un mode de réalisation de l'invention adapté pour atteindre des très hautes densités cellulaires (1 à $3 \times 10^9$ cellules/ml), le milieu de culture nommé « Milieu batch» du Tableau 1 ci-dessous peut être utilisé pour le démarrage de la culture en continu, et la solution d'alimentation du Tableau 1 est utilisée pour alimenter les fermenteurs en continu.

**Tableau 1** : Exemple de milieux de culture utiles pour la culture des souches de Aurantiochytrium, selon l'étape i) du procédé de culture.

| | Milieu pied | Solution alimentation |
|---|---|---|
| Source carbone | 1 à 1,6 CMol | 3,7 CMol |
| NH4⁺ | 1,5 à 7,5 g/L | 5 à 10 g/L |
| Sel marin | 5 à 30 g/L | 10 à 30 g/L |
| Ca | 25 à 70 mg/L | 100 à 200 mg/L |
| Mn | 1 à 10 mg/L | 4 à 20 mg/L |
| Zn | 0,1 à 1 mg/L | 0,5 à 2 mg/L |
| S | 0,5 à 1,5 g/L | 2 à 4 g/L |
| Co | 0,1 à 1 mg/L | 0,5 à 2 mg/L |
| Mo | 0,01 à 0,5 mg/L | 0,05 à 1,5 mg/L |
| Cu | 0,05 à 0,3 mg/L | 0,25 à 0,8 mg/L |
| Ni | 0,02 à 0,2 mg/L | 0,1 à 0,5 mg/L |
| K | 0,5 à 2 g/L | 3 à 5 g/L |
| Mg | 70 à 300 mg/L | 250 à 750 mg/L |
| P | 0,3 à 1 g/L | 1,3 à 2,5 g/L |
| Fe | 5 à 10 mg/L | 15 à 25 mg/L |
| Thiamine | 15 à 50 mg/L | 50 à 150 mg/L |
| Vitamine B12 | 0,2 à 0,7 mg/L | 0,7 à 2 mg/L |
| Panthoténate | 0,05 à 0,15 mg/L | 15 à 45 mg/L |
| Agent stabilisant (type EDTA) | A fixer par le préparateur | A fixer par le préparateur |

Le « milieu pied» correspond au milieu qui peut être utilisé pour le démarrage de la culture en continu. Ensuite « la solution d'alimentation » peut être utilisée pour alimenter le fermenteur en continu.

Selon le même mode de réalisation, la solution nutritive utilisée dans l'étape de maturation, l'étape ii), contient une source de carbone (par exemple du glucose ou du glycérol), une source d'azote (par exemple du $(NH_4)_2SO_4$, une source de phosphate (par exemple du $KH_2PO_4$). De manière préférée, le ratio entre le C (carbone), le N (l'azote) et le P (le phosphate) (C/N/P) est 530 :11 :1 avec toujours la possibilité d'avoir jusqu'à aux fois plus ou moins d'azote (N), et/ou jusqu'à deux fois plus ou moins de phosphate (P), par exemple les ratios suivants peuvent être utilisés 530:22:1,530:5,5:1; 530:22:0,5; 530:11:2; 530:22:2; 530:5,5:0,5; 530:11:0,5; 530:5,5:2.

Les gammes de concentrations données pour le milieu utilisé pour la pré-culture, la culture discontinue et le milieu d'alimentation de la culture continue sont spécifiquement adaptés à l'espèce Aurantiochytrium mangrovei, notamment, la souche FCC 1324.

De manière générale, lorsque la concentration en biomasse dans le fermenteur de tête a atteint un équilibre, entre environ 5 et 100 g/L, de préférence entre environ 10 et 70 g/L en concentration de biomasse, le soutirage du fermenteur de tête sert à alimenter de manière continue et successive les différents fermenteurs de maturation fonctionnant en mode semi-continu. La culture des cellules est ainsi maintenue en conditions de mixotrophie dans lesdits fermenteurs (étape de « maturation »), en vue de la production de DHA et/ou de caroténoïde(s), en particulier de canthaxanthine et/ou de l'astaxanthine.

Ainsi, de manière générale, l'alimentation des fermenteurs de maturation selon l'étape ii) démarre quand le fermenteur de tête est en régime permanent à une densité cellulaire stable. De manière générale, pour *Aurantiochytrium* cette densité cellulaire pourrait être entre $10^7$ et $10^9$ cellules par mL.

L'alimentation des fermenteurs de maturation selon l'étape ii) se fait (en général) de manière successive, c'est-à-dire que l'on alimente un fermenteur n de maturation avec le soutirage du fermenteur de tête avant de passer au fermenteur de maturation suivant, n+1.

Le volume d'inoculum utilisé pour inoculer le fermenteur de maturation peut représenter entre 10 et 70 % du volume final de la suspension cellulaire dans le fermenteur de maturation, préférentiellement entre 20 et 60 %, plus préférentiellement entre 30 et 50 %.

De manière générale, une fois que le volume d'inoculum souhaité a été atteint dans le fermenteur de maturation n, on alimente le deuxième (n+1) fermenteur de maturation (ou « maturateur »), puis on alimente le troisième (n+2) fermenteur de maturation, etc.

Selon un mode de réalisation, la culture dans le fermenteur de maturation est arrêtée, puis le fermenteur est vidé quand la production de DHA et/ou de caroténoïde(s) atteint le niveau désiré, par exemple, lorsque le titre en DHA est entre 5 et 30 g/L et/ou le titre en caroténoïde entre 0,1 et 10 mg/g de biomasse sèche.

La vidange se fait de manière conventionnelle vers des outils permettant le traitement de la biomasse en vue de l'extraction de la ou des molécule(s) d'intérêt. Après avoir été vidé, le fermenteur de maturation est nettoyé, généralement par un équipement spécifique qui est intégré aux cuves des fermenteurs, puis est stérilisé.

Par exemple (voir le Tableau 2 et le schéma selon la Figure 1), avec un temps de culture dans les fermenteurs de maturation de 72h, le premier jour, J, le fermenteur de maturation 1 est rempli et les autres fermenteurs de maturation sont en attente; à J+ 1, le fermenteur de maturation 2 est rempli (culture N+2) et le fermenteur de maturation 1 est en cours de culture; à J+ 2, le fermenteur de maturation 3 est rempli et les fermenteurs de maturation 1 et 2 sont en cours de culture; à J+ 3, le fermenteur de maturation 1 est nettoyé, le fermenteur de maturation 4 est rempli et les fermenteurs de maturation 2 et 3 sont en cours de culture.

**Tableau 2**

| | Fermenteur de maturation 1 | Fermenteur de maturation 2 | Fermenteur de maturation 3 | Fermenteur de maturation 4 |
|---|---|---|---|---|
| **Jour J** | Culture N | Attente | Attente | Attente |
| **Jour J+1** | Culture N | Culture N+1 | Attente | Attente |
| **Jour J+2** | Culture N | Culture N+1 | Culture N+2 | Attente |
| **Jour J+3** | Nettoyage et stérilisation | Culture N+1 | Culture N+2 | Culture N+3 |
| **Jour J+4** | Culture N+4 | Nettoyage et stérilisation | Culture N+2 | Culture N+3 |
| **Jour J+5** | Culture N+4 | Culture N+5 | Nettoyage et stérilisation | Culture N+3 |

Le nombre de fermenteurs de maturation alimentés par le fermenteur de tête est supérieur à 2, et, de manière générale, ce nombre est limité seulement par l'espace disponible pour stocker lesdits fermenteurs et leurs équipements annexes. En général, le nombre de fermenteurs de maturation est de 2 à 10, de préférence de 2 à 6.

Pour la culture *d'Aurantiochytrium* l'étape de culture en mode semi-continu est de manière générale effectuée pendant 24 à 200 h dans chaque fermenteur, de préférence pendant 48 h à 96 h, plus préférentiellement 72 h à 96 h.

La composition du milieu de culture pour l'étape ii) permet de maintenir une croissance résiduelle, tout en favorisant l'accumulation de DHA et/ou de caroténoïde(s), en particulier de canthaxanthine et/ou de l'astaxanthine. Par « croissance résiduelle », on entend une multiplication des cellules à un taux de croissance compris entre 1 et 90 % du taux de croissance de l'étape i) mais qui permet d'améliorer la productivité en biomasse et en molécule(s) d'intérêt.

Pour la production de DHA par des *Aurantiochytrium* FCC1324, le taux de croissance en culture continue est de 0,05 à 0,20 h$^{-1}$, de préférence de 0,10 h$^{-1}$.

Le taux de croissance caractérise l'accroissement de la population au cours du temps. Le taux de croissance ($\mu$) est calculé selon la formule :

$$\mu = (\ln N2 - \ln N1) / (t2 - t1)$$

où N1 = quantité de biomasse au temps t1, N2 : quantité de biomasse au temps t2. La quantité de biomasse peut être exprimée en densité optique, densité cellulaire ou masse sèche. Les temps t1 et t2 sont exprimés en heure. La valeur de $\mu$ est exprimée en h$^{-1}$ (1/heure).

Le milieu de culture utilisé pour la fermentation selon l'étape ii) de culture en semi-continu est choisi pour orienter préférentiellement le métabolisme des cellules cultivées vers l'accumulation de DHA et/ou de caroténoïde(s), en particulier de canthaxanthine et/ou de l'astaxanthine. Cela est réalisé lors d'une limitation en azote. Par limitation en azote, on entend une concentration en azote insuffisante dans le milieu de culture pour assurer une croissance normale des cellules, et qui se traduit par une modification du métabolisme favorisant l'accumulation du carbone sous forme de lipides. Cette modification du métabolisme peut également être obtenue en augmentant le rapport C/N (rapport de la concentration de carbone sur celle d'azote assimilable).

Toutefois, le milieu de culture contient une source de carbone organique, qui permet une croissance en mixotrophie ou hétérotrophie. Typiquement, pour une culture de protiste, le carbone organique est présent à une concentration de 10 à 90 g/l (de 55mM à 500mM).

En général, pour la culture selon l'étape ii) de maturation (pour la production de DHA (et éventuellement des caroténoïdes) par la souche *Aurantiochytrium,* le milieu de culture (reconstitué à partir du volume de la suspension cellulaire issue du fermenteur de tête et du volume de solution d'alimentation concentrée) comprend de 100 à 200 g/L de glucose, environ 5 à 10g/L d'ammonium et 1 à 2 g/L de phosphate.

La méthode de culture selon un mode de réalisation de l'invention se fait en conditions de mixotrophie et/ou en conditions d'hétérotrophie, c'est-à-dire que l'étape i) peut se faire indépendamment en conditions d'hétérotrophie ou de mixotrophie. De manière générale l'étape ii) se fait en mixotrophie. Selon un mode de réalisation, l'étape i) se fait en hétérotrophie et l'étape ii) en mixotrophie. Selon un autre mode de réalisation, les étapes i) et ii) se font en mixotrophie.

L'étape ii) se fait à une température plus basse que l'étape i). Les inventeurs ont constaté que, selon ce mode de réalisation, la quantité de lipides, notamment de DHA, produite est augmentée par rapport à une culture où l'étape ii) est réalisée à la même température que l'étape i). En effet, selon le procédé d'invention, la température optimale de croissance est supérieure à la température optimale de production de DHA. L'étape ii) est réalisée à 1 à 8 degrés de moins que la température à laquelle l'étape i) est réalisée. Par exemple, l'étape i) de la culture peut être menée à 25°C, tandis que l'étape b) peut être menée à 18°C, 19°C, 20°C, 21 °C, 22°C ou 23°C. Par exemple, l'étape i) de la culture peut être menée à 22°C, tandis que l'étape b) peut être menée à 18°C, 19°C, 20°C ou 21 °C. Par exemple, l'étape i) de la culture peut être menée à 27°C, tandis que l'étape b) peut être menée à 20°C, 21 °C, 22°C, 23°C, 24°C, 25°C ou 26°C. Une température basse favorise également l'accumulation de pigments.

Selon un autre aspect de l'invention, des conditions d'éclairage sont adapté à maximiser le rendement en biomasse, en acides gras, notamment en DHA et des caroténoïdes. Il est apparu qu'un éclairement variable et/ou discontinu des cultures, en particulier dans la mise en oeuvre d'une culture en mode mixotrophe, avait un impact favorable sur le développement des protistes et permettait d'accroître la productivité de celles-ci, notamment en ce qui concerne leur production de lipides et de caroténoïdes. Sans être lié par la théorie, l'inventeur estime qu'un apport discontinu et/ou variable de lumière aux protistes a pour effet de provoquer un « stress » favorable à la croissance et à la synthèse des lipides ainsi qu'à la synthèse des caroténoïdes. Ce phénomène pourrait s'expliquer, en partie, par le fait que dans la nature, les protistes ont tendance à accumuler des réserves lipidiques et en caroténoïdes pour résister aux contraintes de leur environnement.

Par éclairement discontinu, il faut entendre un éclairement ponctué par des périodes d'obscurité. Les périodes d'obscurité peuvent occuper plus d'un quart du temps, de préférence la moitié du temps ou plus, durant lequel les algues sont cultivées.

Selon un aspect préféré de l'invention, l'éclairement est discontinu et plus préférentiellement sous forme de flashs. Un flash, au sens de l'invention, est un éclairement lumineux de courte durée, c'est-à-dire de moins de 30 minutes.

La durée peut être de moins de 15 minutes, de préférence de moins de 5 minutes ou plus préférentiellement encore de moins de 1 minute. Selon certains modes de réalisation de l'invention, la durée du flash peut être de moins d'une seconde. Par exemple, le durée du flash peut être 1/10 d'une seconde, ou 2/10 d'une seconde, ou 3/10 d'une seconde, ou 4/10 d'une seconde ou 5/10 d'une seconde, ou 6/10 d'une seconde, ou 7/10 d'une seconde, ou 8/10 d'une seconde, ou 9/10 d'une seconde. L'éclairement lumineux, ou le flash, est généralement d'une durée supérieure à 15 secondes. Elle est généralement comprise entre 5 secondes et 10 minutes, de préférence entre 10 secondes et 2 minutes, plus préférentiellement entre 20 secondes et 1 minute. Ces durées de flash sont appropriées pour des apports de lumière de « basse fréquence » ;

Selon ce dernier mode de réalisation, ou le régime d'illumination (l'apport de la lumière) est de « basse fréquence », le nombre de flashs est compris entre environ 2 et 3600 par heure. Il peut être, par exemple, compris entre 100 et 3600 flashs par heure. Il peut être également compris entre 120 et 3000, ou entre 400 et 2500, ou entre 600 et 2000, ou entre 800 et 1500 flashs par heure. Il peut être également compris entre 2 et 200, préférentiellement entre 10 et 150, plus préférentiellement entre 15 et 100, et plus préférentiellement encore entre 20 et 50 par heure.

Selon un mode de réalisation, un flash a une durée entre 1/150000 seconde et 1/1000 seconde. Ces durées de flash sont appropriées pour des régimes d'illumination de « haute fréquence » ; c'est-à-dire avec des fréquences de flash de 150kHz à 1kHz respectivement.

Selon ce mode de réalisation ou le régime d'illumination (l'apport de la lumière) est de « haute fréquence », les flashs peuvent avoir lieu entre $3,6 \times 10^5$ et $5,4 \times 10^9$ fois par heure. Dans ce cas la variation de la lumière est dite d'avoir une fréquence d'entre 1kHz et 150kHz, c'est-à-dire entre 1000 et 150 000 flashes par seconde. L'apport de lumière selon ce dernier mode de réalisation de l'invention est nommé « haute fréquence ».

Le nombre de flashs par heure est choisi en fonction de l'intensité et de la durée des flashs (voir ci-dessous). En général, l'intensité de la lumière apportée sous forme de flashs est entre 5 et 1000 $\mu$mol. m$^{-2}$. s$^{-1}$, de préférence entre 5 et 500 $\mu$mol. m$^{-2}$. s$^{-1}$, ou 50 et 400 $\mu$mol. m$^{-2}$. s$^{-1}$, et plus préférentiellement entre 150 et 300 $\mu$mol. m$^{-2}$. s$^{-1}$. 1 $\mu$mol. m$^{-2}$. s$^{-1}$ correspond à 1$\mu$E m$^{-2}$. s$^{-1}$ (Einstein), unité souvent utilisée dans la littérature.

Selon un autre mode de l'invention, l'éclairement peut être variable, ce qui signifie que l'éclairement n'est pas interrompu par des phases d'obscurité, mais que l'intensité lumineuse varie au cours du temps. Cette variation de l'intensité de lumière est régulière et peut être périodique ou cyclique. Selon l'invention, on peut aussi procéder à un apport lumineux alliant des phases d'éclairement continues et discontinues.

Selon l'invention, quelles que soient les conditions d'éclairement, l'intensité lumineuse apportée aux algues en culture, exprimée en micromoles de photons par seconde par mètre carré ($\mu$mol. m$^{-2}$. s$^{-1}$), varie au moins une fois dans une même heure. L'amplitude de cette variation d'intensité de lumière généralement comprise entre 5 et 1000, ou entre 50 et 800, ou entre 100 et 600 $\mu$mol. m$^{-2}$. s$^{-1}$. L'intensité de la lumière peut également varier entre 5 et 400 $\mu$mol. m$^{-2}$. s$^{-1}$. De préférence, l'amplitude de la variation d'intensité de lumière est entre 70 et 300 $\mu$mol. m$^{-2}$. s$^{-1}$ et plus préférentiellement entre 100 et 200 $\mu$mol. m$^{-2}$. s$^{-1}$.

Ladite intensité lumineuse peut atteindre successivement, en conditions d'éclairement variable, par exemple, les valeurs 50 $\mu$mol. m$^{-2}$. s$^{-1}$ et 100 $\mu$mol. m$^{-2}$·s$^{-1}$, ou 5 et 400 $\mu$mol. m$^{-2}$·s$^{-1}$, ou 50 et 800 $\mu$mol. m$^{-2}$. s$^{-1}$ plusieurs fois chaque heure. Ladite intensité lumineuse peut atteindre successivement, de préférence, les valeurs 50 et 200 $\mu$mol. m$^{-2}$. s$^{-1}$. Alternativement, en conditions d'éclairement discontinu, ladite intensité lumineuse peut atteindre successivement, plusieurs fois dans l'heure, par exemple, les valeurs 0 et 50 $\mu$mol. m$^{-2}$. s$^{-1}$, les valeurs 0 et 100 $\mu$mol. m$^{-2}$. s$^{-1}$ ou préférentiellement encore les valeurs 0 et 200 $\mu$mol. m$^{-2}$. s$^{-1}$. Elle peut également atteindre successivement, plusieurs fois dans l'heure, par exemple, les valeurs 0 et 300 $\mu$mol. m$^{-2}$. s$^{-1}$, les valeurs 0 et 600 $\mu$mol. m$^{-2}$. s$^{-1}$, les valeurs 0 et 800 $\mu$mol. m$^{-2}$. s$^{-1}$ ou encore les valeurs 0 et 1000 $\mu$mol. m$^{-2}$. s$^{-1}$.

Selon un mode de l'invention et quelles que soient les conditions d'éclairement, l'intensité de la lumière apportée à la culture varie en fonction de la densité cellulaire. Plus la culture devient dense, plus la lumière peut être intense. La densité cellulaire est le nombre de cellules par ml et est mesurée selon les techniques connues de l'homme de l'art.

Au stade initial de la culture, quand la densité cellulaire est entre environ $10^6$ et $5 \times 10^6$ cellules par ml, l'intensité lumineuse peut être entre 5 et 15 $\mu$mol. m$^{-2}$. s$^{-1}$, de préférence, entre 5 et 10 $\mu$mol. m$^{-2}$. s$^{-1}$. Quand la culture atteint une densité entre $10^7$ et $10^8$ cellules par ml, l'intensité lumineuse peut être augmentée jusqu'à entre 15 et 200 $\mu$mol. m$^{-2}$. s$^{-1}$, par exemple, de préférence, entre 20 et 50 $\mu$mol. m$^{-2}$. s$^{-1}$. Quand la culture, au stade final, atteint une densité entre $10^8$ et $10^9$ cellules par ml, l'intensité lumineuse peut être augmentée jusqu'à entre 50 et 700 $\mu$mol. m$^{-2}$. s$^{-1}$, par exemple, de préférence, entre 50 et 150 $\mu$mol. m$^{-2}$. s$^{-1}$.

Selon certains modes de réalisation, par exemple, quand la durée des flashs est par exemple de moins d'une minute, ou moins d'une seconde, l'intensité de la lumière peut être plus importante par rapport aux valeurs citées ci-dessus. Au stade initial de la culture, quand la densité cellulaire est entre environ $10^6$ et $5 \times 10^6$ cellules par ml, l'intensité lumineuse peut être entre 5 et 200 $\mu$mol. m$^{-2}$. s$^{-1}$, de préférence, entre 5 et 100 $\mu$mol. m$^{-2}$. s$^{-1}$. Quand la culture atteint une densité entre $10^7$ et $10^8$ cellules par ml, l'intensité lumineuse peut être augmentée jusqu'à entre 30 et 500 $\mu$mol. m$^{-2}$. s$^{-1}$, par exemple, de préférence, entre 50 et 400 $\mu$mol. m$^{-2}$. s$^{-1}$. Quand la culture, au stade final, atteint une densité entre $10^8$ et $10^9$ cellules par ml, l'intensité lumineuse peut être augmentée jusqu'à entre 100 et 1000 $\mu$mol. m$^{-2}$. s$^{-1}$ par

exemple, de préférence, entre 200 et 500 $\mu$mol. m$^{-2}$. s$^{-1}$.

Selon un mode de l'invention, la quantité de lumière apportée à la culture dans l'heure reste entre certaines valeurs. Elle est comprise entre environ 2000 et 600 000, de préférence entre 2000 et 300 000 $\mu$mol. m$^{-2}$. Elle peut être comprise entre environ 4000 et 200 000 $\mu$mol. m$^{-2}$ par heure.

Selon un mode de l'invention, la culture est illuminée avec 30 flashs par heure, chaque flash ayant une durée de 30 secondes et une intensité de 10 $\mu$mol. m$^{-2}$. s$^{-1}$. Ce dernier donne un apport total de lumière par heure de 9000 $\mu$mol. m$^{-2}$. Selon un autre mode de l'invention, la culture est illuminée avec 20 flashs par heure, chaque flash ayant une durée de 30 secondes et une intensité de 20 $\mu$mol. m$^{-2}$. s$^{-1}$. Ce dernier donne un apport total de lumière par heure de 12 000 $\mu$mol. m$^{-2}$. Selon un autre mode de l'invention, la culture est illuminée avec 45 flashs par heure, chaque flash ayant une durée de 15 secondes et une intensité de 5 $\mu$mol. m$^{-2}$. s$^{-1}$, ce qui donne un apport total de lumière par heure de 3375 $\mu$mol. m$^{-2}$.

Selon un autre mode de l'invention, la culture est illuminée avec 120 flashs par heure, chaque flash ayant une durée de 10 secondes et une intensité de 200 $\mu$mol. m$^{-2}$. s$^{-1}$, ce qui donne un apport total de lumière par heure de 240 000 $\mu$mol. m$^{-2}$.

Selon un autre mode de réalisation de l'invention, la culture est illuminée avec une fréquence de 50kHz, c'est-à-dire 1.8 x10$^8$ flashs par heure, chaque flash ayant une durée de 1/100 000 secondes et une intensité de 200 $\mu$mol. m$^{-2}$. s$^{-1}$, ce qui donne un apport total de lumière par heure de 36 000 $\mu$mol. m$^{-2}$

Comme décrit pour l'intensité lumineuse ci-dessus, et selon un mode de l'invention, la quantité de lumière apportée à la culture par heure peut varier en fonction de la densité cellulaire. Au stade initial de la culture, quand la densité cellulaire est de 10$^5$ et 5 x 10$^5$ cellules par ml, l'apport total de lumière dans l'heure est généralement compris entre environ 1500 et 8000, de préférence 1500 et 6000 $\mu$mol. m$^{-2}$, de préférence encore entre 2000 et 5000 $\mu$mol. m$^{-2}$. Quand la culture atteint une densité entre 10$^6$ et 10$^7$ cellules par ml, l'apport total de lumière dans l'heure peut être augmenté jusqu'à entre 6000 et 67 000 $\mu$mol. m$^{-2}$, de préférence entre 6000 et 50 000, et de préférence encore entre 12 000 et 45 000 $\mu$mol. m$^{-2}$, par exemple. Au stade final de la culture, à une densité cellulaire entre 10$^7$ et 10$^8$ cellules par ml, l'apport total de lumière dans l'heure peut être augmenté jusqu'à entre 45 000 et 300 000, par exemple, de préférence, entre 45 000 et 200 000 $\mu$mol. m$^{-2}$, et par exemple, de préférence encore, entre 50 000 et 150 000 $\mu$mol. m$^{-2}$.

Selon un mode de l'invention, au stade initial de la culture (à une densité cellulaire entre 10$^5$ et 5 x10$^5$ cellules par ml), la culture est illuminée avec 30 flashs par heure, chaque flash ayant une durée de 30 secondes et une intensité entre 5 et 10 $\mu$mol. m$^{-2}$. s$^{-1}$, ce qui donne un apport total de lumière par heure de 2250 $\mu$mol. m$^{-2}$ à 4500 $\mu$mol. m$^{-2}$. Puis, au stade intermédiaire (à une densité cellulaire entre 10$^6$ et 10$^7$ cellules par ml), la culture est illuminée avec 30 flashs par heure, chaque flash ayant une durée de 30 secondes et une intensité entre 15 et 50 $\mu$mol. m$^{-2}$. s$^{-1}$, ce qui donne un apport total de lumière par heure de 13 500 à 45 000 $\mu$mol. m$^{-2}$. Puis au stade final de la culture (à une densité cellulaire entre 10$^7$ et 10$^8$ cellules par ml), la culture est illuminée avec 30 flashs par heure, chaque flash ayant une durée de 30 secondes et une intensité entre 50 et 150 $\mu$mol. m$^{-2}$. s$^{-1}$, ce qui donne un apport total de lumière par heure de 45 000 à 135 000 $\mu$mol. m$^{-2}$.

Selon un mode de l'invention, par exemple, quand la durée des flashs est par exemple de moins d'une minute, ou moins d'une seconde, au stade initial de la culture (à une densité cellulaire entre 10$^5$ et 5 x 10$^5$ cellules par ml), la culture est illuminée avec 30 flashs par heure, chaque flash ayant une durée de 10 secondes et une intensité entre 50 et 100 $\mu$mol. m$^{-2}$. s$^{-1}$, ce qui donne un apport total de lumière par heure de 15 000 $\mu$mol. m$^{-2}$ à 30 000 $\mu$mol. m$^{-2}$. Puis au stade intermédiaire (à une densité cellulaire entre 10$^6$ et 10$^7$ cellules par ml), la culture est illuminée avec 50 flashs par heure, chaque flash ayant une durée de 10 secondes et une intensité entre 200 et 300 $\mu$mol. m$^{-2}$. s$^{-1}$, ce qui donne un apport total de lumière par heure de 100 000 à 150 000 $\mu$mol. m$^{-2}$. Puis, au stade final de la culture (à une densité cellulaire entre entre 10$^7$ et 10$^8$ cellules par ml), la culture est illuminée avec 120 flashs par heure, chaque flash ayant une durée de 10 secondes et une intensité entre 350 et 450 $\mu$mol. m$^{-2}$. s$^{-1}$, ce qui donne un apport total de lumière par heure de 420 000 à 540 000 $\mu$mol. m$^{-2}$.

L'apport de lumière dans les cultures peut être obtenu par des sources de lumière réparties autour de la paroi externe des fermenteurs. Une horloge déclenche ces lampes pour des temps d'éclairement définis. Selon un mode de réalisation préféré, l'éclairage des cultures est assuré par un dispositif d'illumination interne au fermenteur. Ainsi, l'efficacité de l'illumination est meilleure par rapport à une configuration où la lumière pénètre par des hublots à partir de sources disposées à l'extérieur. Les dispositifs d'illumination peuvent être disposés sur l'ensemble tournant muni de pales ou sur des pièces tubulaires plongeant dans la masse à traiter, ou encore au fond ou au plafond de la cuve. Selon un mode de réalisation préféré de l'invention, le dispositif d'illumination est situé sur les contre-pales à l'intérieur du fermenteur. Un tel dispositif est décrit dans la demande de brevet français N°1353641. Le fermenteur est ainsi muni d'une pluralité de sources d'illumination portées par des contre-pales, ces dernières ayant pour fonction d'empêcher la formation d'un vortex au sein de la biomasse sous l'action de l'ensemble tournant de brassage. Ces sources d'illumination sont de préférence encapsulées, partiellement ou complètement dans au moins une partie de ces contre pales, dans une matière compatible avec la biomasse et en une épaisseur permettant de diffuser ladite lumière vers l'intérieur de la cuve.

Une matière particulièrement bien adaptée est le polysulfone qui combine une bonne compatibilité avec les normes alimentaires (y compris les normes américaines de la Food and Drug Administration, ou FDA en abrégé), un bon coefficient de transfert thermique (permettant une évacuation de la chaleur vers la masse de micro-organismes et un caractère semi-transparent permettant une bonne transmission de la lumière, si le matériau a une épaisseur choisie entre 1 mm et 5 cm ; en outre, ce matériau conserve ses propriétés après un éventuel traitement thermique de stérilisation ou de nettoyage avec des détergents ou de l'acide. Si l'on modifie la combinaison de caractéristiques souhaitées, d'autres matériaux peuvent être choisis, par exemple le polyuréthane, le polypropylène, un matériau acrylique ou un polycarbonate.

De manière préférée, les sources d'illumination sont des diodes électro-luminescentes (LED en abrégé); il s'agit de sources de lumière bien maîtrisées, aussi bien dans leur mise en oeuvre que dans leurs applications. De telles sources peuvent avoir des spectres d'émission très divers, puisqu'il y a des LED blanches (simulant la lumière solaire), mais aussi des LED à gamme spectrale réduite (par exemple centrées sur une lumière rouge, bleue ou verte). De telles sources d'illumination génèrent moins de chaleur que des bulbes ou des ampoules ; elles ont en outre des dimensions suffisamment petites pour pouvoir être implantées sur les faces des contre-pales sans induire de surépaisseur nuisant à la fonction principale de ces plaques.

Le spectre des sources d'illumination est avantageusement dans le domaine du visible, mais peut aussi, en fonction des besoins, être à l'extérieur de ce domaine visible, par exemple dans les UV (par exemple pour des applications impliquant une stérilisation), ou dans les infra-rouges (par exemple, dans des applications visant à générer de la chaleur au sein de la masse à traiter).

Les sources d'illumination peuvent avoir des régimes de commande (on parle aussi de régimes de contrôle ou de pilotage) très variés

Ainsi qu'a pu le constater le déposant, le fait que les souches ainsi cultivées présentent de bonnes aptitudes à croître en mode mixotrophe, en présence d'une lumière discontinue et/ou variable, prédispose lesdites souches à une production plus élevée d'acides gras polyinsaturés et en caroténoïdes, notamment en DHA et en canthaxanthine et/ou en astaxanthine.

Le procédé de culture selon l'invention permet de produire des lipides et des caroténoïdes.

Dans ce cas, le procédé selon certains modes de réalisation de l'invention comporte en outre les étapes suivantes :

a) la culture en mode mixotrophe d'une ou plusieurs souches de la classe *Labyrinthulomycete,* en particulier du genre *Aurantiochytrium* dans des conditions mixotrophes, en particulier en présence d'un éclairement discontinu et/ou variable au cours du temps, l'éclairement présentant des variations d'intensité dont l'amplitude est comprise entre 5 $\mu$mol. m$^{-2}$. s$^{-1}$ et 1000, de préférence entre 5 et 400 $\mu$mol. m$^{-2}$. s$^{-1}$, ces variations ayant lieu entre 2 et 3600, de préférence 5-400 fois par heure,

b) une étape de maintien de ladite culture sur plusieurs générations, en présence d'un substrat carboné organique dans le milieu de culture, et éventuellement

c) une étape de récupération des protistes ainsi cultivés.

Par étape de récupération, on entend plus particulièrement l'isolement de la ou des souches dont le nombre de cellules s'est accru le plus au cours desdites générations.

d) une étape de récupération de la matière hydrophobe des protistes, et éventuellement

e) l'extraction du DHA (acide docosahexaénoïque) et des caroténoïdes, notamment de la canthaxanthine et/ou d'astaxanthine de la matière hydrophobe récupérée.

La matière hydrophobe comporte en effet les lipides et les caroténoïdes.

Le procédé de culture selon l'invention peut s'appliquer également à toute espèce du genre *Aurantiochytrium,* capable de croître dans les conditions mixotrophes selon l'invention, et capable de produire du DHA et des caroténoïdes.

L'invention a donc également pour but l'optimisation de la production de biomasse, ainsi que la production de lipides et de caroténoïdes, notamment de DHA, et/ou de canthaxanthine et/ou d'astaxanthine via la culture de protistes du genre *Aurantiochytrium* à caractère mixotrophe, de préférence cultivés ou sélectionnés selon les procédés visés précédemment, puis la récupération des protistes ainsi cultivés pour en extraire le contenu hydrophobe, en particulier les lipides dont le DHA et les caroténoïdes dont la canthaxanthine et l'astaxanthine.

Selon les modes de réalisation du procédé de production de DHA et/ou de la canthaxanthine et/ou en astaxanthine, décrits précédemment, les quantités de biomasse obtenus sont supérieures à 100g/L, de préférence supérieures à 110g/L ou plus préférentiellement supérieures à 140g/L. Les lipides sont présent dans cette biomasse à un taux supérieur à 40%, voire 50%. En générale le DHA représente plus de 15%, voir 20%, voir 30%, voire plus 35% des acides gras. La canthaxanthine et/ou l'astaxanthine peut représenter entre 0,01 %, et 0,2 % en poids sur le poids total de matière sèche.

Les méthodes d'extraction sélective des lipides dont le DHA sont connues de l'homme du métier et sont, par exemple, décrites par [Bligh, E.G. et Dyer, W.J. (1959) ; A rapid method of total lipid extraction and purification, Can. J. Biochem. Physiol., 37:911-917].

Les méthodes d'extraction et d'analyse des caroténoïdes, dont la lutéine, sont connues de l'homme du métier et sont, par exemple, décrites par Wright et al. (1991) (S.W. Wright, S.W. Jeffrey, R.F.C. Mantoura, C.A. Llewellyn, T. Bjornland, D. Repeta, N. Welschmeyer : Improved HPLC method for the analysis of chlorophylls and carotenoids from marine phytoplankton. Marine ecology progress sériés : Vol. 77 : 183-196, 1991).

L'invention porte également sur les protistes de la classe *Labyrinthulomycete,* notamment de la famille *Labyrinthulaceae* et *Thraustochytriaceae*, en particulier du genre *Aurantiochytrium,* susceptibles d'être obtenus selon le procédé de l'invention tel que précédemment décrit. Ces protistes sont enrichis en acides gras polyinsaturés et en caroténoïdes. Les lipides totaux de tels protistes représentent généralement plus de 40 %, ou plus de 50 %, ou plus de 60 % en poids de la matière sèche. De manière générale, ils peuvent avoir un titre de DHA de 10 g/L à 20 g/L. La canthaxanthine et/ou l'astaxanthine contenue par de tels protistes, selon un mode de l'invention, peut représenter plus de 0,1 %, ou plus de 0,15 %, ou plus de 0,2 % en poids sur le poids total de matière sèche. Les protistes selon un mode de réalisation de l'invention peuvent avoir ainsi une productivité (quantité de produit d'intérêt produit, par litre de culture, par heure) de canthaxanthine et/ou de l'astaxanthine de 0,05 mg/L/h, ou plus de 1 mg/L/h, ou plus de 1,5 mg/L/h. Ils peuvent avoir également avoir une productivité de DHA d'au moins 0,1 g/L/h, de préférence 0,2 g/L/h DHA, et plus préférentiellement d'au moins 0,3 g/L/h DHA.

## Exemple 1

Les cultures *d'Aurantiochytrium* sont réalisées dans des fermenteurs (bioréacteurs) de 1 à 2 L utiles avec automates dédiés et supervision par station informatique. Le système est régulé en pH via l'ajout de base (solution d'hydroxyde de sodium à 2N) et/ou d'acide (solution d'acide sulfurique à 1N). La température de culture est fixée à 26 °C. L'agitation est réalisée grâce à 3 mobiles d'agitation placés sur l'arbre selon la configuration de Rushton (hélices tripales à pompage descendant). La pression d'oxygène dissous est régulée dans le milieu tout au long de la culture, par la vitesse d'agitation (250-600 t/min), le débit d'air (0,25- 1 vvm), voire le débit d'oxygène (0,1-0,5 vvm). Les paramètres de régulation, intégrés dans l'automate de supervision, permettent de maintenir une $pO_2$ constante à 15 %. Le bioréacteur est équipé d'un système luminaire externe entourant la cuve transparente. L'intensité ainsi que les cycles de lumière sont contrôlés par un automate dédié et supervisé par station informatique.

Les réacteurs sont inoculés avec une pré-culture réalisée sur table d'agitation (140 t/min) en enceinte thermostatée (26 °C) et éclairée entre 100 et 200 $\mu$E. Les pré-cultures et cultures en bioréacteurs sont réalisées dans le milieu Verduyn modifié (sels marins 15 g/L, $(NH_4)_2SO_4$ 3g/L , $KH_2PO_4$ 1 g/L, $MgSO_4 \cdot 7H_20$, 0,5 g/L, $Na_2EDTA$ 24 mg/L, $ZnSO_4 \cdot 7H_2O$ 3 mg/L, $MnCl_2 \cdot 2H_2O$ 3 mg/L, $Na_2MoO_4 \cdot 2H_2O$ 0,04 mg/L, $FeSO_4 \cdot 7H_2O$ 10mg/L, pantothénate 3,2 mg/L, hydrochlorure de thiamine 9,5 mg/L, vitamine B12 0,15 mg/L). Le substrat carboné utilisé pour la culture en mixotrophie classique, en hétérotrophie et en mixotrophie flash (lumière variable/discontinue) en bioréacteur est du glucose à des concentrations entre 300 mM et 1 M.

Suivi des cultures :

La concentration en biomasse totale est suivie par mesure de la masse sèche (filtration sur filtre GF/F, Whatman, puis séchage en étuve, à 105 °C, pendant 24 h minimum avant pesée).

Concernant la quantification des lipides totaux, $10^8$ cellules ont été extraites. Les méthodes d'extraction des lipides sont connues de l'homme du métier.

Pour la quantification des caroténoïdes, et notamment la canthaxanthine et de l'astaxanthine, $10^8$ cellules ont été extraites. Les méthodes d'extraction et d'analyse des caroténoïdes, dont la canthaxanthine et de l'astaxanthine, sont connues de l'homme du métier.

Eclairement (pour la culture en mixotrophie) :

La culture est illuminée avec 30 flashs par heure, chaque flash ayant une durée de 30 secondes et une intensité de 200 $\mu$mol. $m^{-2}$. $s^{-1}$.

L'apport de lumière dans les cultures en bioréacteur a été obtenu par des lampes LED réparties autour de la paroi externe du fermenteur. Une horloge déclenche ces LED pour des temps d'éclairement ou des pulses (pour la culture en mixotrophie flash avec lumière discontinue).

**Tableau 3** Résultats de l'Exemple 1(n=3) :

| | Masse Sèche (g/L) | Lipides totaux (% de la MS) | % DHA (des acides gras totaux) | Astaxanthine (mg/g de MS) |
|---|---|---|---|---|
| Mixotrophie Flash | 115 +/- 2 | 56 +/- 3,6 | 18,3 +/- 2,8 | 1,1 +/-0,1 |
| Hétérotrophie | 112,2 +/- 1,6 | 44,5 +/- 2,9 | 19,1 +/-2,5 | 0 |

**Exemple 2**

La production d'une huile riche en DHA et en canthaxanthine par la souche FCC 1324 du genre *Aurantiochytrium* est décrite ci-dessous.

Etape i) :

Les cultures *d'Aurantiochytrium* sont réalisées dans des fermenteurs (bioréacteurs) de 1 à 2 L utiles avec automates dédiés et supervision par station informatique. Le système est régulé en pH via l'ajout de base (solution d'hydroxyde de sodium à 2N) et/ou d'acide (solution d'acide sulfurique à 1N). La température de culture est fixée à 26 °C. L'agitation est réalisée grâce à 3 mobiles d'agitation placés sur l'arbre selon la configuration de Rushton (hélices tripales à pompage descendant). La pression d'oxygène dissous est régulée dans le milieu tout au long de la culture, par la vitesse d'agitation (250-600 t/min), le débit d'air (0,25-1 vvm), voire le débit d'oxygène (0,1-0,5 vvm). Les paramètres de régulation, intégrés dans l'automate de supervision, permettent de maintenir une $pO_2$ constante à 15 %. Le bioréacteur est équipé d'un système luminaire interne fixé sur les contre-pales. L'intensité, ainsi que les cycles de lumière, sont contrôlés par un automate dédié et supervisé par station informatique. La culture est illuminée avec 60 flashs par heure, chaque flash ayant une durée de 20 secondes et une intensité de 100 $\mu$mol. m$^{-2}$. s$^{-1}$.

Les réacteurs sont inoculés avec une pré-culture réalisée sur table d'agitation (140 t/min) en enceinte thermostatée (26 °C) et éclairée entre 100 et 200 $\mu$E. Pré-cultures et cultures en bioréacteurs sont réalisées dans le milieu Verduyn modifié (sels marins 15 g/L, $(NH_4)_2SO_4$ 3g/L, $KH_2PO_4$ 1 g/L, $MgSO_4 \cdot 7H_2O$, 0,5 g/L, $Na_2EDTA$ 24 mg/L, $ZnSO_4 \cdot 7H_2O$ 3 mg/L, $MnCl_2 \cdot 2H_2O$ 3 mg/L, $Na_2MoO_4 \cdot 2H_2O$ 0,04 mg/L, $FeSO_4 \cdot 7H_2O$ 10mg/L, pantothénate 3,2 mg/L, hydrochlorure de thiamine 9,5mg/L, vitamine B12 0,15 mg/L). Le substrat carboné utilisé est du glucose à des concentrations entre 60 et 200 g/L.

L'alimentation en continu avec du milieu frais est réalisée avec un taux de dilution d'environ 0,08 à 0,15 h$^{-1}$ et avec un milieu concentré entre 10 et 15 fois la concentration initiale de chaque élément. L'homme du métier saura déterminer comment mettre en oeuvre la culture continue et calculer le débit d'alimentation, ainsi que déterminer quand le régime permanent est atteint.

Une fois le régime permanent atteint, le soutirage de ce fermenteur continu peut servir à alimenter le fermenteur de maturation de l'étape b).

Etape ii) :

Les cultures sont réalisées dans des fermenteurs (bioréacteurs) de 10 à 20 L utiles avec automates dédiés et supervision par station informatique. Les systèmes de régulation ainsi que le paramétrage de ces derniers sont, en tout point, similaires à ceux de l'étape i).

Les réacteurs sont inoculés avec environ 50 % du volume total de culture avec le soutirage de l'étape i) ; et en même temps, la cuve est également alimentée au même débit avec un milieu concentré 2 fois, et qui permet d'obtenir la composition finale suivante : sels marins 15 g/L ; glucose entre 60 et 120 g/L; $(NH_4)_2SO_4$ à 0,8 gL; $KH_2PO_4$ 1 g/L ; $MgSO_4 \cdot 7H_2O$, 0,5 g/L, $Na_2EDTA$ 24 mg/L, $ZnSO_4 \cdot 7H_2O$ 3 mg/L, $MnCl_2 \cdot 2H_2O$ 3 mg/L, $Na_2MoO_4 \cdot 2H_2O$ 0,04 mg/L, $FeSO_4 \cdot 7H_2O$ 10mg/L, pantothénate 3,2 mg/L, hydrochlorure de thiamine 9,5 mg/L, vitamine B12 0,15 mg/L.

La culture est illuminée avec 60 flashs par heure, chaque flash ayant une durée de 20 secondes et une intensité de 500 $\mu$mol. m$^{-2}$. s$^{-1}$.

Suivi des cultures :

La concentration en biomasse totale est suivie par mesure de la masse sèche (filtration sur filtre GF/F, Whatman, puis séchage en étuve, 105 °C, pendant 24h minimum avant pesée).

Concernant la quantification des lipides totaux, environ $10^8$ cellules ont été extraites. Les méthodes d'extraction des lipides sont connues de l'homme du métier.

Pour la quantification des caroténoïdes, et notamment la canthaxanthine, $10^8$ cellules ont été extraites. Les méthodes d'extraction et d'analyse des caroténoïdes, dont la canthaxanthine, sont connues de l'homme du métier.

**Tableau 4** : Résultats de l'Exemple 2 (n=3)

| Masse Sèche (g/L) | Lipides totaux (% de la MS) | % DHA | Canthaxanthine (mg/g de MS) |
|---|---|---|---|
| 155 +/- 3,3 | 55 +/- 2,8 | 18,0 +/- 3,0 | 2,1 +/- 0,3 |

**Exemple 3**

La production en continu d'une biomasse à haute densité cellulaire pauvre en matière grasse (5 à 10%) puis l'accumulation d'acides gras par maturation de cette biomasse

Etape i): Culture continue pour la production de biomasse

Les cultures d'Aurantiochytrium sont réalisées dans des réacteurs de 1 à 2 L de volume utile avec automates dédiés et supervision par station informatique. Le système est régulé en pH via l'ajout de base (solution d'hydroxyde de sodium à 2N) et/ou d'acide (solution d'acide sulfurique à 1N). La température de culture est fixée à 26 °C. L'agitation est réalisée grâce à 3 mobiles d'agitation : 1 turbine Rushton à 6 pâles droites positionnée à l'extrémité inférieure de l'arbre d'agitation au-dessus du sparger et 2 hélices tripâles placées sur l'arbre d'agitation (distance inter-mobile = 1.5 X le diamètre d'une hélice).

La pression d'oxygène dissous est régulée dans le milieu tout au long de la culture, par la vitesse de rotation de l'arbre d'agitation (250-600 t/min), la ventilation par l'air (0,25-1 vvm) ou d'oxygène (0,1-0,5 vvm). Les paramètres de régulation, intégrés dans l'automate de supervision, permettent de maintenir une $pO_2$ constante à 15 %.

Le fermenteur est équipé d'un système luminaire interne fixé sur les contre-pâles.

L'intensité et la fréquence des cycles d'illumination sont contrôlées par un automate dédié et supervisé par station informatique. La culture est illuminée avec 60 flashs par heure, chaque flash ayant une durée de 20 secondes et une intensité de 100 µmol. m-2. s-1.

La pré-culture est réalisée sur table d'agitation (140 t/min) en enceinte thermostatée (26 °C) et éclairée entre 100 et 200 µE. Le réacteur est inoculé à 1% à partir de la pré-culture et conduit en mode discontinu.

Une fois le substrat contenu dans le milieu initial consommé et la densité cellulaire souhaitée atteinte, le soutirage du milieu de culture d'une part et l'apport de la solution d'alimentation d'autre part sont mis en route. Le régime permanent est atteint après au moins 5 temps de séjour. Une fois le régime permanent stabilisé, les paramètres opératoires (débit de ventilation, puissance dissipée, débit d'alimentation, pH, volume de moût), la concentration résiduelle en substrat dans le moût, ainsi que la concentration et la composition macromoléculaire de la biomasse produite sont constants.

Pour la souche FCC 1324, la composition de la solution d'alimentation choisie et le taux de dilution sélectionné permettent de produire un volume de moût équivalent à 2,4 fois le volume du fermenteur chaque jour.

Le régime est considéré stable au bout de 50 heures de culture. Une fois le régime stabilisé, la concentration résiduelle en glucose dans le moût se stabilise vers 0 g/L, la densité cellulaire se stabilise autour de $3 \times 10^9$ cellules/mL, ce qui correspond à environ 65 g/L de biomasse, la teneur en matière grasse dans la biomasse se stabilise autour de 5% (gMatière Grasse/gMatière Sèche). La fermentation en mode continu a été maintenue pour une durée totale de 890 heures, dont 815 heures en régime stabilisé.

Pour cet exemple le taux de dilution par la solution d'alimentation est fixé à la moitié du taux de croissance maximum de la souche sur le milieu utilisé et dans les conditions de culture utilisées.

Le débit de soutirage du milieu est adapté afin de prendre en compte le débit de la solution d'alimentation mais aussi le volume de liquide correcteur de pH.

La composition du milieu de culture pour le démarrage en batch de la culture est détaillée dans le Tableau 1.

La solution d'alimentation de la culture continue contient 15g/L de sel de mer commercial (ex Sels INSTANT OCEAN), 110 g/L de glucose, et contient des concentrations en oligo-éléments, macro-éléments et vitamines équivalentes à environ 3 fois celles contenues dans le milieu utilisé pour la pré-culture et la culture discontinue. Des autres compositions pour la solution d'alimentation sont possible, comme détaillée dans le Tableau 1.

La concentration en biomasse totale est suivie par mesure de la masse sèche (filtration sur filtre GF/F, Whatman, puis séchage en étuve, 105 °C, pendant 24h minimum avant pesée).

Concernant la quantification des lipides totaux, environ $10x^8$ cellules ont été extraites. Les méthodes d'extraction des lipides sont connues de l'homme du métier.

Pour la quantification des caroténoïdes, et notamment la canthaxanthine, $10x^8$ cellules ont été extraites. Les méthodes d'extraction et d'analyse des caroténoïdes, dont la canthaxanthine, sont connues de l'homme du métier.

Tableau 5. Caractéristiques du régime permanent obtenu en culture continue.

| | | |
|---|---|---|
| Glucose résiduel | 0.1 ± 0.02 | g/L |
| Densité cellulaire | 3.109 +/- 0,5.109 | cellules/mL |
| Masse sèche | 65 +/- 5 | g/L |
| Teneur en acide gras | 7% +/- 3% | % |

Etape ii) de maturation

Le moût produit en culture continue présente donc une haute densité cellulaire d'une biomasse contenant très peu de lipides (5 à 10%). Cette biomasse est transférée dans une cuve de maturation dans des conditions de culture où elle accumule très rapidement des acides gras. (65 G/L en 24 heures). Ceci permet de produire une forte concentration de biomasse riche en acide gras.

Dans cet exemple, plusieurs essais de maturation sont effectués à partir du moût issu du fermenteur conduit en mode chemostat. Les cultures sont réalisées dans des fermenteurs (bioréacteurs) de 3 à 5 L utiles avec automates dédiés et supervision par station informatique. Les systèmes de régulation ainsi que le paramétrage de ces derniers sont, en tout point, similaires à ceux de l'étape 1.

Dans le cas des essais de maturation décrits dans cet exemple, le volume prélevé dans la culture continue est prélevé en une fois. Dans le cas d'une fermentation industrielle, ce transfert se fait progressivement par le soutirage du milieu de culture.

Dans la cuve de maturation, la solution nutritive avec un ratio C/N/P de 530 :11 :1 est progressivement ajoutée au volume prélevé.

La solution nutritive est ajoutée dans la cuve de maturation à chaque fois que la source de carbone (ici le glucose) est épuisée.

Le suivi de la culture discontinue alimentée, réalisée en cuve de maturation, est réalisé selon des protocoles en tout point identiques à ceux de l'étape i). Les résultats sont donnés dans le Tableau 5.

**Tableau 6 :**

| Matièresèche, acidesgraset DHAproduitsen 24heureset en48heureslorsdela maturation à 25°C etpH6,5 (5 répétitions) | 24Heures deculture | 48Heures deculture |
|---|---|---|
| Matière Sèche | 160 g/L (+/- 2g/l) | 185 g/l (+/-5g/l) |
| Acides Gras | 67 g/L (+/-2g/l) | 85 g/l (+/-5g/l) |
| DHA | 16,5 g/L(+/-0,5g/l) | 21 g/L (+/-2g/l) |
| Astaxanthine | 0,05 mg/g MS (+/-0,01) | 0,07 mg/g MS (+/-0,01) |

**Revendications**

**1.** Procédé de production de DHA et/ou de canthaxanthine et/ou de l'astaxanthine, comprenant l'étape suivante :

a) la culture en mode mixotrophe d'une ou plusieurs souches du genre *Aurantiochytrium* **caractérisé en ce que** l'étape a) de la culture comprend

- Une étape i) de croissance en conditions de mixotrophie ou d'hétérotrophie et
- Une étape ii) d'accumulation de DHA et/ou de canthaxanthine et/ou d'astaxanthine en conditions de mixotrophie à une température entre 15 et 25°C,

**caractérisé en ce que** la souche du genre *Aurantiochytrium* correspond à la souche FCC 1324, déposée auprès de la CCAP (Culture Collection of Algae and Protozoa), sous le numéro d'accession CCAP 4062/1 et l'étape ii) se fait à une température plus basse de 1 à 8 degrés que l'étape i).

**2.** Procédé selon la revendication 1 **caractérisé en ce que** l'étape i) est réalisé en mode continu dans un fermenteur et l'étape ii) comprend l'alimentation en continu et successivement de n fermenteurs fonctionnant en mode semi-continu, n étant un nombre entier égal ou supérieur à 2, par les cellules produites dans l'étape i) et leur culture en conditions de mixotrophie.

**3.** Procédé selon la revendication 1 ou 2, **caractérisé en ce que** la culture en mode mixotrophe se fait dans des conditions d'éclairement discontinu et/ou variable au cours du temps, l'éclairement présentant des variations d'intensité dont l'amplitude est comprise entre 5 $\mu$mol. m$^{-2}$. s$^{-1}$ et 1000 $\mu$mol. m$^{-2}$. s$^{-1}$, ces variations ayant lieu entre 2 et 3600 fois par heure.

**4.** Procédé selon la revendication 2, **caractérisé en ce que** l'amplitude des variations d'intensité est comprise entre 70 et 300 $\mu$mol. m$^{-2}$. s$^{-1}$, et de préférence, entre 100 et 200 $\mu$mol. m$^{-2}$. s$^{-1}$.

**5.** Procédé selon l'une quelconque des revendications 1 à 4, **caractérisé en ce que** l'apport de lumière s'effectue sous forme de flashs.

**6.** Procédé selon la revendication 5, **caractérisé en ce qu'**un flash a une durée comprise entre 1/10 seconde et 10 minutes, de préférence 5 secondes et 10 minutes, de préférence encore entre 10 secondes et 2 minutes, plus préférentiellement entre 20 secondes et 1 minute.

**7.** Procédé selon la revendication 5 **caractérisée en ce qu'**un flash a une durée entre 1/150000 seconde et 1/1000 seconde.

**8.** Procédé selon la revendication 5 ou la revendication 6, **caractérisé en ce que** le nombre de flashs est entre 5 et 3600, de préférence entre 10 et 150, préférentiellement entre 15 et 100, et plus préférentiellement entre 20 et 50 fois par heure.

**9.** Procédé selon la revendication 7, **caractérisé en ce que** le nombre de flashs est entre $3,6 \times 10^5$ et $5,4 \times 10^9$ par heure.

**10.** Procédé selon l'une des revendications 1 à 9 **caractérisé en ce que** la culture s'effectue en présence d'un substrat carboné organique à une concentration de 100 mM à 1,5 M, de préférence de 200 mM à 1,2 M, plus préférentiellement de 250 mM à 1 M, et encore plus préférentiellement de 300 mM à 600 mM d'un substrat carboné organique, le substrat carboné organique étant choisi parmi le saccharose, le glycérol, le glucose et les dérivés de cellulose et un mélange de ces molécules.

**11.** Procédé selon l'une quelconque des revendications 1 à 10, **caractérisé en ce qu'**il comprend en outre les étapes suivantes :

b) une étape de maintien de ladite culture sur plusieurs générations en présence d'un substrat carboné organique dans le milieu de culture, et éventuellement
c) une étape de récupération des cellules ainsi cultivés, et éventuellement
d) une étape de récupération de la matière hydrophobe, et éventuellement
e) l'extraction du DHA (acide docosahexaénoïque) et de canthaxanthine et/ou d'astaxanthine de la matière hydrophobe récupérée.

**12.** Procédé selon l'une quelconque des revendications 1 à 11, **caractérisé en ce que** l'éclairement des cultures est assuré par un dispositif d'illumination interne au fermenteur, de préférence, situé sur les contre-pales à l'intérieur du fermenteur.

**Patentansprüche**

**1.** Verfahren zur Produktion von DHA und/oder von Canthaxanthin und/oder von Astaxanthin, umfassend den folgenden Schritt:

a) das Kultivieren im mixotrophen Modus von einem oder mehreren Stämmen der Gattung *Aurantiochytrium,* **dadurch gekennzeichnet, dass** der Schritt a) des Kultivierens umfasst

- einen Wachstumsschritt i) unter Mixotrophie- oder Heterotrophiebedingungen und
- einen Akkumulationsschritt ii) von DHA und/oder von Canthaxanthin und/oder von Astaxanthin unter Mixotrophiebedingungen bei einer Temperatur zwischen 15 und 25 °C,

**dadurch gekennzeichnet, dass** der Stamm der Gattung *Aurantiochytrium* dem Stamm FCC 1324 entspricht, hinterlegt bei der CCAP (Culture Collection of Algae and Protozoa) unter der Hinterlegungsnummer CCAP 4062/1 und
der Schritt ii) bei einer um 1 bis 8 Grad niedrigeren Temperatur als der Schritt i) durchgeführt wird.

2. Verfahren nach Anspruch 1, **dadurch gekennzeichnet, dass** der Schritt i) im kontinuierlichen Modus in einem Fermenter durchgeführt wird und der Schritt ii) die kontinuierliche und aufeinanderfolgende Versorgung von n Fermentern, die im halbkontinuierlichen Modus arbeiten, umfasst, wobei n eine Ganzzahl gleich oder über 2 ist, mit Zellen, die in Schritt i) produziert wurden, und ihr Kultivieren unter Mixotrophiebedingungen.

3. Verfahren nach Anspruch 1 oder 2, **dadurch gekennzeichnet, dass** das Kultivieren im mixotrophen Modus unter Beleuchtungsbedingungen erfolgt, die im Laufe der Zeit diskontinuierlich und/oder variabel sind, wobei die Beleuchtung Stärkevariationen aufweist, deren Amplitude zwischen 5 $\mu$mol.m$^{-2}$.s$^{-1}$ und 1000 $\mu$mol.m$^{-2}$.s$^{-1}$ liegt, wobei diese Variationen zwischen 2 und 3600 Mal pro Stunde stattfinden.

4. Verfahren nach Anspruch 2, **dadurch gekennzeichnet, dass** die Amplitude der Stärkevariationen zwischen 70 und 300 $\mu$mol.m$^{-2}$.s$^{-1}$ und vorzugsweise zwischen 100 und 200 $\mu$mol.m$^{-2}$.s$^{-1}$ liegt.

5. Verfahren nach einem der Ansprüche 1 bis 4, **dadurch gekennzeichnet, dass** die Lichtzufuhr in Form von Blitzen erfolgt.

6. Verfahren nach Anspruch 5, **dadurch gekennzeichnet, dass** ein Blitz eine Dauer hat, die zwischen 1/10 Sekunde und 10 Minuten, vorzugsweise 5 Sekunden und 10 Minuten, noch vorzugsweiser zwischen 10 Sekunden und 2 Minuten, in noch bevorzugterer Weise zwischen 20 Sekunden und 1 Minute liegt.

7. Verfahren nach Anspruch 5, **dadurch gekennzeichnet, dass** ein Blitz eine Dauer zwischen 1/150000 Sekunde und 1/1000 Sekunde hat.

8. Verfahren nach Anspruch 5 oder Anspruch 6, **dadurch gekennzeichnet, dass** die Anzahl der Blitze zwischen 5 und 3600, vorzugsweise zwischen 10 und 150, in bevorzugter Weise zwischen 15 und 100, und in noch bevorzugterer Weise zwischen 20 und 50 Mal pro Stunde liegt.

9. Verfahren nach Anspruch 7, **dadurch gekennzeichnet, dass** die Anzahl der Blitze zwischen 3,6 x 10$^5$ und 5,4 x 10$^9$ pro Stunde liegt.

10. Verfahren nach einem der Ansprüche 1 bis 9, **dadurch gekennzeichnet, dass** das Kultivieren bei Anwesenheit eines organischen kohlenstoffhaltigen Substrats in einer Konzentration von 100 mM bis 1,5 M, vorzugsweise von 200 mM bis 1,2 M, in bevorzugterer Weise von 250 mM bis 1 M und in noch bevorzugterer Weise von 300 mM bis 600 mM eines organischen kohlenstoffhaltigen Substrats erfolgt, wobei das organische kohlenstoffhaltige Substrat aus der Saccharose, dem Glycerol, der Glucose und den Zellulosederivaten und einem Gemisch dieser Moleküle ausgewählt ist.

11. Verfahren nach einem der Ansprüche 1 bis 10, **dadurch gekennzeichnet, dass** es ferner die folgenden Schritte umfasst:

b) einen Schritt des Aufrechterhaltens der Kultur über mehrere Generationen bei Anwesenheit eines organischen kohlenstoffhaltigen Substrats im Kulturmilieu, und eventuell
c) einen Wiedergewinnungsschritt der derart kultivierten Zellen und eventuell
d) einen Wiedergewinnungsschritt des hydrophoben Materials, und eventuell
e) die Extraktion der DHA (Docosahexaensäure) und von Canthaxanthin und/oder von Astaxanthin aus dem wiedergewonnenen hydrophoben Material.

12. Verfahren nach einem der Ansprüche 1 bis 11, **dadurch gekennzeichnet, dass** die Beleuchtung der Kulturen von einer fermenterinternen Beleuchtungsvorrichtung gewährleistet wird, die sich vorzugsweise auf den Gegenschaufeln im Inneren des Fermenters befindet.

**Claims**

1. Process for the production of DHA and/or canthaxanthin and/or astaxanthin, comprising the following step:

    a) culture in mixotrophic mode of one or more strains of the genus *Aurantiochytrium* **characterized in that** step a) of the culture comprises

        - A step i) of growth under mixotrophic or heterotrophic conditions and
        - A step ii) of accumulation of DHA and/or canthaxanthin and/or astaxanthin under mixotrophic conditions at a temperature between 15 and 25°C,

    **characterized in that** the strain of the genus *Aurantiochytrium* is strain FCC 1324, deposited at the CCAP (Culture Collection of Algae and Protozoa), under accession number CCAP 4062/1 and step ii) is carried out at a temperature 1 to 8 degrees lower than step i).

2. Process according to claim 1, **characterized in that** step i) is carried out in continuous mode in a fermenter and step ii) comprises the continuous and successive feeding of *n* fermenters operating in semi-continuous mode, n being an integer equal to or greater than 2, with the cells produced in step i) and their culture under mixotrophic conditions.

3. Process according to claim 1 or 2, **characterized in that** the culture in mixotrophic mode is carried out under illumination conditions that are discontinuous and/or variable over time, the illumination having variations in intensity the amplitude of which is comprised between 5 $\mu$mol.m$^{-2}$.s$^{-1}$ and 1000 $\mu$mol.m$^{-2}$.s$^{-1}$, these variations taking place between 2 and 3600 times per hour.

4. Process according to claim 2, **characterized in that** the amplitude of the variations in intensity is comprised between 70 and 300 $\mu$mol.m$^{-2}$.s$^{-1}$, and preferably between 100 and 200 $\mu$mol.m$^{-2}$.s$^{-1}$.

5. Process according to any one of claims 1 to 4, **characterized in that** the light is supplied in the form of flashes.

6. Process according to claim 5, **characterized in that** a flash has a duration comprised between 1/10 second and 10 minutes, preferably 5 seconds and 10 minutes, more preferably between 10 seconds and 2 minutes, more preferentially between 20 seconds and 1 minute.

7. Process according to claim 5, **characterized in that** a flash has a duration between 1/150000 second and 1/1000 second.

8. Process according to claim 5 or claim 6, **characterized in that** the number of flashes is comprised between 5 and 3600, preferably between 10 and 150, preferentially between 15 and 100, and more preferentially between 20 and 50 times per hour.

9. Process according to claim 7, **characterized in that** the number of flashes is between $3.6 \times 10^5$ and $5.4 \times 10^9$ per hour.

10. Process according to one of claims 1 to 9, **characterized in that** the culture is carried out in the presence of an organic carbon-containing substrate at a concentration of 100 mM to 1.5 M, preferably 200 mM to 1.2 M, more preferably 250 mM to 1 M, and even more preferably 300 mM to 600 mM of an organic carbon-containing substrate, the organic carbon-containing substrate being selected from sucrose, glycerol, glucose and cellulose derivatives and a mixture of these molecules.

11. Process according to any one of claims 1 to 10, **characterized in that** it further comprises the following steps:

    b) a step of maintaining said culture over several generations in the presence of an organic carbon-containing substrate in the culture medium, and optionally
    c) a step of recovery of the thus cultured cells, and optionally
    d) a step of recovery of the hydrophobic matter, and optionally

e) the extraction of DHA (docosahexaenoic acid) and canthaxanthin and/or astaxanthin from the recovered hydrophobic matter.

**12.** Process according to any one of claims 1 to 11, **characterized in that** the illumination of the cultures is provided by an illumination device internal to the fermenter, preferably located on the baffles inside the fermenter.

Figure 1

Figure 2

# Figure 3

(A)                                    (B)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2009209014 A
- EP 2559342 A
- FR 1353641

**Littérature non-brevet citée dans la description**

- **CHATDUMRONG.** *KasetsartJ. (Nat.Sci.),* 2007, vol. 41 (2), 324-334
- **W.K. HONG et al.** Production of lipids containing high levels of docosahexaenoic acid by a newly isolated microalga, Aurantiochytrium sp. KRS101. *Appl. Biochem. Biotechnol.,* 2011, vol. 164 (8), 1468-80
- **YOKOYAMA, R ; HONDA, D.** Taxonomic rearrangement of the genus Schizochytrium sensu lato based on morphology, chemotaxonomic characteristics, and 18S rRNA gene phylogeny (Thraustochytriaceae, Labyrinthulomycetes): emendation for Schizochytrium and erection of Aurantiochytrium and Oblongichytrium gen. *Mycoscience,* Novembre 2007, vol. 48, 199-211
- Culture Collection of Algae and Protozoa. Dunstaffnage Marine Laboratory. Scottish Association for Marine Science
- **BLIGH, E.G. ; DYER, W.J.** A rapid method of total lipid extraction and purification. *Can. J. Biochem. Physiol.,* 1959, vol. 37, 911-917
- **S.W. WRIGHT ; S.W. JEFFREY ; R.F.C. MANTOURA ; C.A. LLEWELLYN ; T. BJORNLAND ; D. REPETA ; N. WELSCHMEYER.** Improved HPLC method for the analysis of chlorophylls and carotenoids from marine phytoplankton. *Marine ecology progress sériés,* 1991, vol. 77, 183-196